(19) **Europäisches Patentamt**
**European Patent Office**
**Office européen des brevets**

(11) **EP 2 096 130 B1**

(12) **EUROPÄISCHE PATENTSCHRIFT**

(45) Veröffentlichungstag und Bekanntmachung des Hinweises auf die Patenterteilung:
**25.04.2012 Patentblatt 2012/17**

(51) Int Cl.:
**C08G 63/64** *(2006.01)* **A61L 17/10** *(2006.01)*

(21) Anmeldenummer: **09002714.5**

(22) Anmeldetag: **26.02.2009**

(54) **Resorbierbares Blockpolymer, Verfahren zu seiner Herstellung und Verwendung**

Reabsorbing block polymer and method for its manufacture and application

Polymère en bloc résorbable et son procédé de fabrication et d'utilisation

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **28.02.2008 DE 102008012620**

(43) Veröffentlichungstag der Anmeldung:
**02.09.2009 Patentblatt 2009/36**

(73) Patentinhaber: **Deutsche Institute für Textil- und Faserforschung**
**Denkendorf Stiftung des öffentlichen Rechts**
**73770 Denkendorf (DE)**

(72) Erfinder:
- **Oberhoffner, Sven, Dr.**
  **71384 Weinstadt (DE)**
- **Müller, Erhard, Dr.**
  **70565 Stuttgart (DE)**
- **Planck, Heinrich, Prof. Dr.**
  **72622 Nürtingen (DE)**

(74) Vertreter: **Patentanwälte**
**Ruff, Wilhelm, Beier, Dauster & Partner**
**Kronenstrasse 30**
**70174 Stuttgart (DE)**

(56) Entgegenhaltungen:
**EP-A- 0 626 404     DE-A1- 19 641 334**

- **LIETZ M ET AL: "PHYSICAL AND BIOLOGICAL PERFORMANCE OF A NOVEL BLOCK COPOLYMER NERVE GUIDE" BIOTECHNOLOGY AND BIOENGINEERING, WILEY & SONS, HOBOKEN, NJ, US, Bd. 93, Nr. 1, 5. Januar 2006 (2006-01-05), Seiten 99-109, XP003005189 ISSN: 0006-3592**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft ein resorbierbares Blockcopolymer der Formel X(BA)$_n$ mit endständigen Hydroxylgruppen, wobei n 2,3 oder 4 ist, X ein Rest eines mehrwertigen Alkohols mit zwei bis vier Hydroxylgruppen ist, B ein Weichsegment aus Caprolacton, Trimethylencarbonat und gegebenenfalls Glykolid ist, wobei die Gesamtmenge an Trimethylencarbonat und Caprolacton im Weichsegment bei 80 bis 100 Gew.-% und die von Glykolid bei 0 bis 20 Gew.-% liegt, A ein Hartsegment aus Caprolacton ist und wobei das Gesamtgewichtsverhältnis von Caprolacton zu Trimethylencarbonat im Blockpolymer im Bereich von 65:35 bis 90:10 liegt, sowie ein Verfahren zu seiner Herstellung und seine Verwendung.

Die vorliegende Erfindung betrifft insbesondere nahezu umesterungsfrei hergestellte Caprolacton-Trimethylencarbonat ABA-Blockcopolymere mit Schmelzpunkten und Schmelzenthalpien nahe dem erzielbaren theoretischen Maximum und deren Verwendung für resorbierbare, medizinische Anwendungen. Ohne die Applikationen einzuschränken, sind insbesondere Nervenleitschienen und Beschichtungen von chirurgischen Nahtmaterialien zu nennen, wobei diese Anwendungsformen ganz oder nur teilweise aus dem erfindungsgemäßen Polymer bestehen können.

Caprolacton und Trimethylencarbonat werden meist in Form von Copolymeren mit beispielsweise Glykolid und/oder Laktid sowie p-Dioxanon zur Herstellung von resorbierbaren Medizinprodukten eingesetzt. Im Bereich der chirurgischen, monofilen Nahtmaterialien befinden sich diese Monomere im Weichsegment der ABA-Blockco- und -terpolymere und dienen dazu, aufgrund ihres geringen Glasumwandlungspunktes und der Amorphität des Weichsegmentes die Biegesteifigkeit zu reduzieren. Die Hartsegmente sind mehrheitlich aus Glykolid gebildet. Als Beispiele seien hier Monosyn der Fa. Aesculap (EP 0835895, US 6048947), Monocryl der Fa. Ethicon (EP 0 441 322 A1, US 475619) und Biosyn der Fa. Tyco (EP 0 626 404 A2, US 688 11, US 204721) genannt. PCL der Fa. Catgut ist ein monofiles Nahtmaterial auf Basis eines statistischen Polycaprolacton-co-laktids mit geringem Comonomeranteil. Als synthetisch resorbierbarer Hautersatz kommt beispielsweise ein statistisches Copolymer aus D,L-Laktid, Caprolacton und Trimethylencarbonat unter dem Handelsnamen Suprathel (EP 1181941, US 6706058) zum Einsatz. Ähnliche Systeme werden als Folie zur Adhäsionsprophylaxe verwendet.

Homopolymere und statistische Copolymere auf Basis von Polycaprolacton werden ferner in Kombination mit Gleitmitteln wie Calciumstearat für die Beschichtung resorbierbarer, multifiler Nahtmaterialien (Handelsnamen Mepfil, Serafit S, Polysorb) eingesetzt. Ebenso zur Beschichtung verwendete Polymere sind statistische Copolymere aus Glykolid und Laktid im Verhältnis 35/65 Gewichtsprozent in Kombination mit Calciumstearat (US 4201216), statistische Copolymere aus Glykolid und Trimethylencarbonat im Zusammensetzungsbereich zwischen 75/25 und 25/75 Prozent (US 4705820) und statistische, nieder- bis mittelmolekulare Copolymere aus Weichmonomeren wie Caprolacton oder Trimethylencarbonat mit Hartmonomeren wie Glykolid oder Laktid und mindestens 5 % Rhizinusöl (US 5371176). Casey et. al. (US 4857602) beschreiben Di- und Triblockcopolymere, gebildet aus Polyethylenoxid-und Polyglykolsäureblöcken. Letztgenannte besitzen Eigenschaften, die denen der Hydrogele nahe kommen.

Resorbierbare Nervenleitschienen werden überwiegend aus Polymeren auf Laktidbasis durch Dip-Coating aus Lösung hergestellt: Odega et. al (Valero-Cabre and Navarro (2002)) verwenden hierzu kristallines Poly-L-Laktid mit dem Nachteil hoher Biegesteifigkeit und zum Teil extrem langer Absorptionszeiten, bedingt durch das Vorhandensein von PLLA-Kristalliten. Letzteres wird durch Dunnen et. al. (J. Biomater. Res. 36 (3) (1997) 337-346) und Pego et. al. (J. Mater. Sci. Mater. Med. 14(9) (2003) 767-773) gelöst, indem Nervenleitschienen aus amorphen Poly-DL-Laktiden hergestellt werden. Allen Laktid-basierten, statistischen Copolymeren gemeinsam ist der deutlich über Raumtemperatur liegende Glasumwandlungspunkt, der zu hohen Biegesteifigkeiten führt. Außerdem wird im Verlauf der Degradation Milchsäure freigesetzt und damit der pH-Wert im Wundbereich erniedrigt. Dies führt zu verstärkter Narbenbildung und kann letztendlich dazu führen, dass die durchtrennten Nervenenden nicht mehr zusammenwachsen oder bereits zusammengewachsene Nerven aufgrund von Kompressionseffekten wieder absterben. Negativ ist auch das nicht zu vernachlässigende Quellvermögen dieser Polymere, das ebenfalls dazu führen kann, dass der Innendurchmesser der Nervenleitröhre verringert und der Nerv dadurch gequetscht wird.

Komplett amorphe und transparente Nervenleitschienen mit geringem Quellvermögen aus L-DL-Laktid-Caprolacton-Copolymeren mit hohem Molekulargewicht werden von Hissink et. al. (US2003/0147934 A1) beansprucht. Der DL-Laktid-Anteil variiert zwischen 51 % und 75 %, die Polymere werden in einer einstufigen Polymerisation synthetisiert. Zur Erzielung hoher Molekulargewichte (i.V. > 4dl/g) und günstiger Monomersequenzen (nicht zu verwechseln mit Segmenten bei Blockpolymeren) sind niedere Polymerisationstemperaturen im Bereich zwischen 100 °C und 130 °C bei Polymerisationsdauern bis zu 40 Tagen notwendig.

Pego et. al. (J. Biomater. Sci. Polym. Ed. 12 (2001) 35-53) beschreiben die statistische, einstufige Polymerisation von Copolymeren aus Caprolacton und Trimethylencarbonat mit Anteilen von 10 - 87 % an Trimethylencarbonat und deren Verarbeitung zu porösen Nervenleitschienen. Die Polymerisationen finden ohne Rühren in einer Ampulle bei 130 °C über einen Zeitraum von 3 Tagen statt. Lediglich die statistischen Copolymere mit 10 - 15 mol-% Diethylencarbonat zeigen Eigenschaftsprofile, die die sinnvolle Weiterverarbeitung zulassen. Als Porenbildner wird von Pego et. al. Natriumchlorid eingesetzt, das anschließend herausgelöst wird (Leaching-Prozeß).

Bei Lietz et. al. (Biotechnology and Bioengineering Vol 93, No. 1 (2006) 99-109) werden Nervenleitschienen aus reinen linearen Polycaprolacton-Trimethylencarbonat-Triblockcopolymeren gebildet. Dabei besteht das Weichsegment der Polymere bei Lietz et .al. aus äquivalenten Anteilen von Trimethylencarbonat und Caprolacton, der Hartsegmentanteil beträgt zwischen 40 % und 70 %. Die Wände dieser flexiblen Nervenleitschienen sind zwecks des Transportes von Metaboliten mikroporös, wobei der cut-off so liegt, dass keine Infiltration von Narben bildenden Zellen möglich ist. Die zur Herstellung dieser Nervenleitschienen benutzten Caprolacton-Trimethylencarbonat-Blockcopolymere zeigen starke Inhomogenitäten innerhalb eines Lots und eine äußerst geringe Reproduzierbarkeit. Die Schmelzenthalpien liegen mit cirka 30 J/g deutlich unter dem theoretisch möglichen Maximum und sind überraschenderweise für beide beschriebene Blockpolymere identisch, obwohl der Hartsegmentanteil bei einem Polymer von 40 % auf 70 % erhöht wurde. Die Blockpolymere sind weich. Bei Raumtemperatur liegt ein nicht unerheblicher Teil der Polymere geschmolzen vor, was die mechanische Festigkeit und die Dimensionsstabilität deutlich beeinträchtigt und die Porenstruktur von Membranen zum kollabieren bringen kann. Eine Verarbeitung der Polymere zu den Nervenleitschienen erfolgt mittels dip-coating.

Es ist ein Ziel der vorliegenden Erfindung, resorbierbare Polymere zur Verfügung zu stellen, die im Lot homogen und reproduzierbar zu synthetisieren sind, die während ihrer Degradation und Absorption im Körper den pH-Wert im Wundbereich nicht oder nur unwesentlich absenken, deren Degradationsprofil durch die prozentuale Zusammensetzung variierbar ist, ohne dadurch das mechanische Eigenschaftsspektrum signifikant zu verändern, die im Bereich zwischen Raum- und Körpertemperatur elastisches Verhalten zeigen, die teilkristallin sind und einen Schmelzpunkt (Maximum DSC-Peak) oberhalb 37 °C besitzen, in gängigen halogenfreien organischen Lösemitteln zur Verarbeitung aus Lösung löslich sind und die sich mittels thermoplastischer Verfahren verarbeiten lassen.

Die Erfindung ist dadurch gekennzeichnet, dass das Blockpolymer eine Schmetzenthalpie von mindestens 32 J/g, insbesondere im Bereich von 32 bis 58 J/g besitzt, wobei die Schmelzenthalpie bei einem Gesamtcaprolactonanteil von 65 Gew.-% eher im unteren Teil des Bereichs und bei einem Caprolactonanteil von 90 Gew.-% eher im oberen Teil des Bereichs liegt.

Durch die Erfindung werden ABA-Blockcopolymere mit A = Hartsegment (gebildet aus Caprolacton) und B = Weichsegment (hydroxterminiertes, statistisches Copolymer aus Trimethylencarbonat und Caprolacton und in einer Sonderform mit bis zu 20 Gew.-% Glykolid) in einer zweistufigen Polymerisation zur Verfügung gestellt, die eine hohe Schmelzenthalpie und schmale Schmelzpeaks in DSC besitzen. Die erfindungsgemäßen Blockpolymere sind bei Raumtemperatur fest. Erreicht wird dies, indem in einer zweiten Polymerisationsstufe, dem Anpolymerisieren des Caprolactons an das hydroxyterminierte Weichsegment, eine nennenswerte Umesterung bzw. Insertion des Caprolactons in das Weichsegment vermieden wird. Dadurch erreichen sowohl die Schmelzpunkte als auch die Schmelzenthalpien, die ein Maß für die Kristallinität des Polymeren darstellen, ihr Maximum und liegen weit über den Werten, die bei den bekannten statistischen Copolymeren oder Blockcopolymeren der gleichen overall-Zusammensetzung erzielbar waren.

Es wurde gefunden, dass die Schmelzenthalpie des erfindungsgemäßen Blockcopolymers je höher ist, je höher der Gesamtcaprolactonanteil im Polymer ist. So kann die Schmelzenthalpie bei einem Caprolacton : Trimethylencarbonat Verhältnis von 65:35 bis 80:20 bei 32 bis 35 J/g oder höher liegen und bei einem Caprolacton-Trimethylencarbonat Verhältnis von 75:25 bis 90:10 bei 45 bis 58 J/g liegen. Auch die Reinheit des Hartsegmentes wirkt sich positiv auf die Eigenschaften des Blockcopolymers und insbesondere auf dessen Schmelzenthalpie aus. Bei einer bevorzugten Ausführungsform der Erfindung ist deshalb der Anteil an Caprolacton-Caprolacton-Bindungen im Hartsegment höher als 80%, insbesondere höher als 90% und liegt bevorzugt bei 92 bis 98%. Auch hier wirkt sich die Höhe des Caprolacton : Trimethylencarbonat Verhältnisses günstig auf die Reinheit des Hartsegmentes aus. Bei einem Caprolacton : Trimethylencarbonat Verhältnis von 65:35 bis 75:25 liegt der Anteil an Caprolacton-Caprolacton-Bindungen im Hartsegment eher bei 80 bis 92% und bei einem Verhältnis von 75:25 bis 90:10 eher bei 92 bis 98 %.

Auch das Verhältnis von Hartsegment : Weichsegment, das indirekt auch das Gesamtverhältnis von Caprolacton : Trimethylencarbonat im Blockpolymer beeinflusst, wirkt sich auf die Eigenschaften und insbesondere die Schmelzenthalpie des Blockcopolymers aus. Bei Blockcopolymeren mit 50 Gew.-% Hartsegmentanteil liegt die Schmelzenthalpie in der Regel bei mindestens 32 J/g. Bei Blockcopolymeren mit Hartsegmentanteil von 60 Gew.-% bei mindestens 37 J/g, bei Hartsegmentanteilen von 70 Gew.-% bei mindestens 45 J/g und bei Hartsegmentanteilen von 70 bis 75 Gew.-% bei 45 bis 58 J/g.

Es wurde weiterhin gefunden, dass auch der Restmonomergehalt möglichst gering sein sollte. Vorzugsweise liegt deshalb der Restmonomergehalt im Blockcopolymer unter 2 Gew.-%. Das erfindungsgemäße Blockcopolymer besitzt insbesondere aufgrund seines kristallinen Hartsegmentanteils und seines amorphen Weichsegmentanteils elastische Eigenschaften, was bevorzugt ist. Weiterhin besitzt es mit Vorteil thermoplastische Eigenschaften, was wiederum auf den harten Hartsegmentanteil zurückzuführen ist. Der Glasübergangspunkt des erfindungsgemäßen Blockcopolymers liegt vorzugsweise maximal bei -45 °C. In der Regel darunter, insbesondere zwischen -45 und -60 °C. Weiterhin besitzt das Blockcopolymer mit Vorteil einen Schmelzpeak (DSC), der im Bereich von 38 bis 57 °C liegt. Die Halbwertsbreite des Schmelzpeaks liegt mit Vorteil in einem engen Schmelzbereich. So liegt die Halbwertsbreite bei einem Weichsegmentanteil von ≤ 30 Gew.-% vorzugsweise bei < 8 °C (Temperaturdifferenz) und bei einem Weichsegmentanteil von 30 bis 40 Gew.-% vorzugsweise bei < 10 °C und bei einem Weichsegmentanteil von 40 bis 50 Gew.-% vorzugsweise < 12 °C.

Im übrigen kann das erfindungsgemäße Blockcopolymer Zusammensetzungen und Eigenschaften besitzen, die aus dem Stand der Technik (insbesondere aus Lietz et al.) bekannt sind. So liegt der Hartsegmentanteil vorzugsweise bei mindestens 50 Gew.-%, insbesondere bei 70 bis 75 Gew.-%. Das Gewichtsverhältnis von Caprolacton : Trimethylencarbonat im Weichsegment kann im Bereich von 30:70 bis 70:30, insbesondere etwa bei 50:50 liegen. Das Gewichtsverhältnis von Hartsegment : Weichsegment im Blockpolymer liegt, wie bereits erwähnt, vorzugsweise zwischen 75:25 und 30:70, insbesondere zwischen 70:30 und 50:50. Das Gesamtverhältnis von Caprolacton : Trimethylencarbonat im Blockpolymer liegt vorzugsweise im Bereich von 75:25 bis 90:10 Gewichtsanteilen.

Die inherente Viskosität, gemessen bei 30 °C in HFIP (Hexafluorisopropanol) und einer Konzentration von 0,5 Gew.-% liegt vorzugsweise im Bereich von 0,7 dl/g bis 1,8 dl/g, insbesondere im Bereich von 1,1 dl/g bis 1,6 dl/g. Der mehrwertige Alkohol (X), der als Initiator bei der Herstellung des Blockcopolymers verwendet wird, ist vorzugsweise ein Alkohol aus der Gruppe: Ethylenglykol, Diethylenglykol, Hexandiol, Glyzerin und Pentraerythrit. Das Molverhältnis von mehrwertigem Alkohol zu den Monomeren Trimethylencarbonat, Caprolacton und gegebenenfalls Glykolid ist gering und liegt mit Vorteil bei 1:250 bis 1:60000, insbesondere bei 1:500 bis 1:50000. Wenn Glykolid vorhanden ist, dann liegt dies bevorzugt im Weichsegment vor. Der Anteil von Glykolid im Weichsegment kann bei einer Gewichtsmenge von 1 bis 20 Gew.-%, vorzugsweise 4 bis 14 Gew.-%, bezogen auf das Gewicht des Weichsegments, betragen. Aufgrund einer nicht immer ganz zu vermeidenden Umesterung ist es auch möglich, dass vernachlässigbare Mengen an Glykolid im Hartsegment vorhanden sein können. Durch geeignete Verfahrensführung lässt sich eine solche unerwünschte Umesterung jedoch weitgehend vermeiden. Insgesamt kann das Weichsegment 24 bis 70 Gew.-% Caprolacton, 24 bis 70 Gew.-% Trimethylencarbonat und 0 bis 20 Gew.-% Glykolid enthalten. Caprolacton und Trimethylencarbonat liegen im Weichsegment vorzugsweise in einem Gewichtsverhältnis von 30:70 bis 45:55 vor. Wie bereits erwähnt, liegt der Gesamtcaprolactonanteil im Blockcopolymer bei 65 bis 90 Gew.-%. Der Gesamtanteil an Trimethylencarbonat im Blockpolymer liegt vorzugsweise bei 10 bis 35 Gew.-%. Falls Glykolid vorhanden ist, liegt der Gesamtgewichtsanteil von Trimethylencarbonat und Glykolid im Blockcopolymer vorzugsweise bei 10 bis 35 Gew.-%.

Das erfindungsgemäße Blockcopolymer ist ein resorbierbares Produkt, das sich besonders für die medizinische Anwendung eignet, insbesondere als Beschichtungsmaterial für medizinische Produkte. Zudem betrifft die Erfindung auch medizinische Produkte, insbesondere Nahtmaterialien, welche das Blockcopolymer gemäß der vorliegenden Erfindung umfassen. Bevorzugt besitzen die medizinischen Produkte, insbesondere Nahtmaterialien, eine Beschichtung aus dem Blockcopolymer nach der Erfindung. Erfindungsgemäß kann es weiterhin vorgesehen sein, dass das Blockcopolymer, insbesondere als Beschichtungsmaterial, beispielsweise für ein Nahtmaterial, in Kombination mit Magnesium-und/oder Calciumsalz von Fettsäuren oder Fettsäureestern, insbesondere solchen der Stearinsäure, vorliegt. Bevorzugt beträgt das Verhältnis aus Blockcopolymer zu Fettsäuresalz zwischen 25 : 75 und 75 : 25 Gew.%, vorzugsweise zwischen 40 : 60 und 60 : 40 Gew.%. Besonders bevorzugt liegt das Blockcopolymer in Kombination mit Calciumsteaoryllactylat vor.

Aufgrund seiner günstigen physikalischen und mechanischen Eigenschaften kann das erfindungsgemäße Blockpolymer auch ein selbständiges Produkt sein. Ein weiterer Aspekt der vorliegenden Erfindung betrifft daher einen Formkörper, welcher ein Blockcopolymer gemäß der vorliegenden Erfindung aufweist. Der Formkörper kann beispielsweise in Form einer Folie oder porösen Membran vorliegen und insbesondere zur Haut- und/oder Wundabdeckung geeignet sein. Das Produkt bzw. der Formkörper ist aber auch als Trägermaterial, insbesondere als drug delivery Material, für medizinische Wirkstoffe und bevorzugt als Trägermaterial für die gesteuerte Geweberegeneration geeignet. Für die letzte Anwendung kann ein vorgeformter beispielsweise vliesförmiger resorbierbarer Träger mit Zellen besiedelt werden. Die Zellen bilden dann entlang des Trägermaterials neues Gewebe aus. Mit fortschreitender Degradation und Resorption des Trägermaterials wird dessen Massenanteil reduziert, bis es vollständig abgebaut ist. Übrig bleibt das nachgewachsene Gewebe. Weiterhin kann chirurgisches Nahtmaterial selbst aus den erfindungsgemäßen Blockcopolymeren gebildet sein. Das erfindungsgemäße Blockpolymer kann aber auch zur Herstellung anderer Produkte Verwendung finden, so kann es in Form eines textilen Materials, eines Schlauches oder eines Schwammes vorliegen. Weiterhin ist das erfindungsgemäße Blockpolymer zur Herstellung von Nervenleitschienen geeignet. So kann mindestens eine äußere Membran einer Nervenleitschiene aus dem Blockpolymer nach der Erfindung bestehen.

Wird das erfindungsgemäße Blockpolymer als Beschichtungsmaterial für Nahtmaterial verwendet, dann kann es mit Vorteil in Kombination mit weiteren Beschichtungsmaterialien für Nahtmaterial vorliegen. Geeignet für eine solche Kombination sind Magnesium- und/oder Calciumsalze von Fettsäuren oder Fettsäureestern, insbesondere solchen der Stearinsäure. Das Verhältnis von Blockcopolymer : Fettsäuresalz liegt vorzugsweise zwischen 25:75 und 75:25 Gew.-%, insbesondere zwischen 40:60 und 60:40 Gew.-%. Besonders geeignet zur Kombination mit dem erfindungsgemäßen Blockcopolymer als Beschichtungsmaterial ist Calciumsteaoryllactylat.

Ein weiterer Aspekt der Erfindung betrifft die Verwendung des Blockcopolymers zur Herstellung von Produkten durch thermoplastische Verarbeitung.

Die Erfindung betrifft auch ein Verfahren zur Herstellung des Blockcopolymers nach der Erfindung. Bei diesem Verfahren werden in einer ersten Reaktionsstufe Trimethylencarbonat, Caprolacton und gegebenenfalls Glykolid unter Verwendung eines zwei- bis vierwertigen Alkohols als Initiator zu einem vollständig amorphen hydroxyterminierten Prepolymer, das das Weichsegment bildet, polymerisiert. Danach wird in einer zweiten Stufe das Caprolacton unter Ausbildung des Hartsegmentes und Bildung des Blockcopolymers anpolymerisiert. Wesentlich ist bei der Bildung des Hartsegmentes, dass die Temperatur während der zweiten Stufe bei maximal 150 °C gehalten wird. Die Polymerisation zur Herstellung des Weichsegmentes wird vorzugsweise vollständig durchgeführt, insbesondere bis zu einem Restmonomergehalt von weniger als 2 Gew.-%. Dadurch kann vermieden werden, dass Restmonomere in unerwünschtem Maße während der zweiten Stufe in das Hartsegment eingebaut werden. Die Reaktionstemperatur während der ersten Stufe, d.h. der Bildung des Weichsegmentes, kann im üblichen Temperaturbereich gehalten werden, insbesondere bei einer Temperatur von 180 bis 220 °C, vorzugsweise 200 bis 210 °C. Wesentlich ist die Absenkung der Temperatur vor Durchführung der zweiten Reaktionsstufe. Hier wird die Reaktionstemperatur vorzugsweise bei 130 bis 150 °C, insbesondere 135 bis 150 °C gehalten. Durch die Temperaturabsenkung dauert die Bildung des Hartsegmentes zwar länger als bei einer höheren Polymerisationstemperatur. Die niedrigere Temperatur ist aber wichtig zur Vermeidung von Umesterungen von Monomeren vom Weichsegment in das Hartsegment. Solche Umesterungen, wie sie als Ergebnis bei den Blockpolymeren nach dem Stand der Technik (vgl. Lietz et al.) gefunden wurden, führen nämlich dazu, dass das Hartsegment mit Trimethylencarbonatmonomeren bzw. gegebenenfalls Glykolidmonomeren durchsetzt ist, was die Kristallinität des Hartsegmentes negativ beeinflusst. Die zweite Reaktionsstufe wird vorzugsweise dann abgebrochen, wenn der Restmonomergehalt unter 2 Gew.-% liegt. Dies wirkt sich insbesondere vorteilhaft auf die erwünschten physikalischen Eigenschaften des Blockcopolymers aus. An sich wäre ein Restmonomergehalt deutlich unter 2% erwünscht. Eine Verlängerung der Reaktionsdauer bis zum Erreichen eines Restmonomergehaltes unter 1 % birgt aber die Gefahr einer verstärkten Umesterung in sich, weshalb hier ein geeigneter Kompromiss getroffen wird.

Das zweistufige Verfahren nach der Erfindung kann ohne vorherige Isolation des Weichsegments vor Beginn der zweiten Stufe durchgeführt werden. Es reicht aus, das Weichsegment auf die Temperatur der zweiten Stufe, d.h. auf maximal 150 °C, abzukühlen oder abkühlen zu lassen. Dann kann die zweite Stufe im selben Gefäß wie die erste Stufe durchgeführt werden. Wird das Weichsegment nach Durchführung der ersten Stufe untersucht, dann besitzt es mit Vorteil eine Glasumwandlungstemperatur von weniger als -30 °C, einen Monomergehalt < 2 Gew.-%, wobei das Gewichtsverhältnis von Caprolacton : Trimethylencarbonat 30:70 bis 70:30 beträgt und das Weichsegment noch bis zu 20 Gew.-% Glykolid enthalten kann. Die Polymerisation des Weichsegments wird vorzugsweise in Gegenwart eines zinnhaltigen Katalysators, insbesondere in Gegenwart von $SnCl_2$ oder 2-Ethylhexansäure-Zinn(II)salz durchgeführt. Die Reaktionsdauer beträgt vorzugsweise 2 bis 8 Stunden. Ein ständiges Rühren während der Polymerisation ist bevorzugt. Die inherente Viskosität des als Prepolymer hergestellten Weichsegments liegt, gemessen in HFIP, bei einer Temperatur von 30 °C und einer Konzentration von 0,5 Gew.-%, vorzugsweise im Bereich zwischen 0,5 dl/g und 1,8 dl/g. Die Viskosität kann durch Variation der Konzentration an Katalysator und Initiator (mehrwertiger Alkohol) eingestellt werden. Das Verhältnis von Gesamtmonomer des Blockcopolymers zum Katalysator (M/K) liegt vorzugsweise im Bereich zwischen 5000 und 120000 mol/mol und insbesondere im Bereich zwischen 10000 und 100000 mol/mol. Das Molverhältnis von mehrwertigem Alkohol (Initiator) zu Katalysator (I/K) liegt mit Vorteil im Bereich zwischen 2 und 20.

Entnimmt man dem Reaktor eine Probe des Weichsegments und lässt diese auf Raumtemperatur abkühlen, so liegt eine plastisch verformbare, zähe, vollständig transparente Masse vor.

Die Umsetzung der hydroxterminierten Weichsegmente zu den ABA-Blockcopolymeren erfolgt, wie bereits erwähnt, ohne vorhergehende Isolierung des Weichsegmentes. Vor Zugabe der Caprolactonmenge, die die Hartsegmente A ausbilden soll, wird die Temperatur des Polymerisationsreaktors auf 130 °C bis 150 °C und bevorzugt auf 135 °C bis 150 °C abgesenkt. Erst nach Erreichen dieser Solltemperatur wird das zuvor abgewogene Caprolacton portionsweise oder in einem Schuss zugegeben. Es wird so viel Caprolacton zur zweiten Polymerisationsstufe zugegeben, dass der Hartsegmentanteil im Blockcopolymer zwischen 30 und 75 Gewichtsprozent und bevorzugt zwischen 50 und 70 Gewichtsprozent beträgt. Der Caprolactonanteil im Blockcopolymer liegt im Bereich zwischen 65 und 90 Gewichtsprozent und bevorzugt zwischen 75 und 90 Gewichtsprozent. In einer besonderen Ausführungsform der Erfindung kann das Blockcopolymer bis zu 14 Gewichtsprozent Glykolid (ausschließlich im Weichsegment gebunden), bezogen auf die Gesamtpolymermasse, enthalten. Die Polymerisation wird unter weiterem ständigen Rühren so lange betrieben, bis der Gesamtumsatz bei mindestens 98 % liegt. Zum Erkennen dieses Punktes wird in regelmäßigen Abständen eine Probe aus dem Reaktor entnommen und mittels 1H-NMR-Spektroskopie untersucht. Ist dieser Punkt erreicht, wird das Polymer vorzugsweise schnellstmöglich aus dem Reaktor abgelassen, da durch weitere Temperatureinwirkung die Umesterung begünstigt und sowohl der Schmelzpunkt als auch die Schmelzenthalpie erniedrigt werden. Die Polymerisationsdauer der zweiten Stufe beträgt in Abhängigkeit von der Katalysator- und Initiatorkonzentration, der Polymerisationstemperatur und dem Weichsegmentanteil in der Regel zwischen 15 Stunden und 8 Tagen und bevorzugt zwischen einem und sechs Tagen. Während beider Stufen wird vorzugsweise gerührt.

Die aus dem Reaktor abgelassenen Polymere erstarren und kristallisieren bei Temperaturen zwischen 55 °C und Raumtemperatur, dabei werden sie aufgrund der Kristallisation opak. Zieht man einen Faden aus einem noch nicht vollständig kristallisierten Polymer, so stellt man fest, dass dieser reversibel elastische Eigenschaften aufweist. Die inhärente Viskosität der ABA-Blockcopolymere, gemessen in HFIP bei 30 °C und einer Konzentration von 0,5 Gewichtsprozent, liegt im Bereich zwischen 0,7 dl/g und 1,8 dl/g und bevorzugt zwischen 0,9 dl/g und 1,6 dl/g. In Abhängigkeit von dem Weichsegmentanteil und der Overall-Zusammensetzung zeigen die Blockcopolymere im DSC (Differential Scanning Calorimetry) ein Maximum des Schmelzpeaks bei Temperaturen zwischen 38 °C und 57 °C und Schmelzenthalpien von mindestens 32 J/g für Blockcopolymere mit 50 % Hartsegmentanteil, mindestens 37 J/g für Hartsegmentanteile von 60 % und mindestens 45 J/g für Hartsegmentanteile von 70 %.

Die erfindungsgemäßen Polymere zeichnen sich insbesondere dadurch aus, dass es gelungen ist, die Polymerisation so zu führen, dass bei der Anpolymerisation der Hartsegmente an die vollständig amorphen, hydroxyterminierten Weichsegmente keine nennenswerte Umesterung und Insertion des zur zweiten Polymerisationsstufe zugegebenen Caprolactons in das Weichsegment stattgefunden hat. Eine nennenswerte Umesterung würde den Blockcharakter des Polymers reduzieren, die Reproduzierbarkeit des Polymerisationsergebnisses dramatisch einschränken, die Kristallinität des Polymers bedeutend erniedrigen und im Extremfall sogar zu einem vollständig amorphen Copolymer führen, das aufgrund seines niedrigen Glasumwandlungspunktes und seiner geringen Enthalpie bei Raumtemperatur eine zähflüssige, honig- bis wachsartige Konsistenz ähnlich der des Weichsegmentes aufweisen würde. Das erfindungsgemäße Polymer weist wegen seines ausgeprägten Blockcharakters folgende Eigenschaften auf: Hohe Flexibilität, Formstabilität und mechanische Festigkeit bei Raumtemperatur sowie einen elastischen Charakter.

Zusätzlich ist aufgrund der chemischen Zusammensetzung die unerwünschte Säurefreisetzung bei der Degradation gegenüber den Laktid und Glykolid basierten Polymeren deutlich erniedrigt. Während letztere bei der hydrolytischen Spaltung Milchsäure und Glykolsäure freisetzen, zerfällt das erfindungsgemäße Polymer hauptsächlich in Caprolacton (die freie Capronsäure ist nicht stabil) und - nach Abspaltung von Kohlendioxid, das vom Körper rasch assimiliert wird - in Propandiol. Der pH-Wert im Wundbereich wird deshalb wenig abgesenkt und es kommt allenfalls zu einer geringen Narbenbildung, was besonders im Fall der Nervenregeneration von entscheidender Bedeutung ist. Falls eine Säurefreisetzung vermieden werden soll, kann das Glykolid im Weichsegment weggelassen werden.

Das Polymer weist weiterhin einen sehr geringen Quellungsgrad auf. Bei der Applikation als Nervenleitschiene ist dies von enormer Bedeutung, da sonst die Gefahr der Quetschung und des Absterbens des nachwachsenden Nervs bestünde.

Aus ökonomischer Sicht ist eine möglichst schnell, jedoch beherrschbar ablaufende Polymerisation erwünscht. Die Polymerisationstemperatur der Weichsegmentpolymerisation wird daher und zum Maximieren des Umsatzes bevorzugt im Bereich zwischen 180 °C und 220 °C und insbesondere zwischen 200 °C und 210 °C durchgeführt. In der zweiten Stufe der Polymerisation (Anpolymerisieren der Hartsegmente) sind Temperaturen unter 130 °C unvorteilhaft: Sie erhöhen die notwendige Polymerisationsdauer bis zum Erreichen eines Umsatzes von mindestens 98 % auf bis zu 12 Tage. Außerdem kann bei langer Reaktionsdauer die Viskosität der Polymerschmelze unerwünscht stark ansteigen.

Betreibt man die Anpolymerisation des Hartsegmentes bei einer Temperatur von mehr als 150 °C, treten insbesondere bei mittleren und höheren Katalysator- und Initiatorkonzentrationen schon unmittelbar oder zumindest innerhalb von 30 Minuten nach Zugabe des Caprolactons Umesterungen auf. Dies führt zu einem Polymer mit eher statistischer denn Blockstruktur und zu reduzierter Kristallinität, im Extremfall sogar zu einem vollständig amorphen Polymer. Auch sind solche Polymerisationen wenig reproduzierbar durchzuführen.

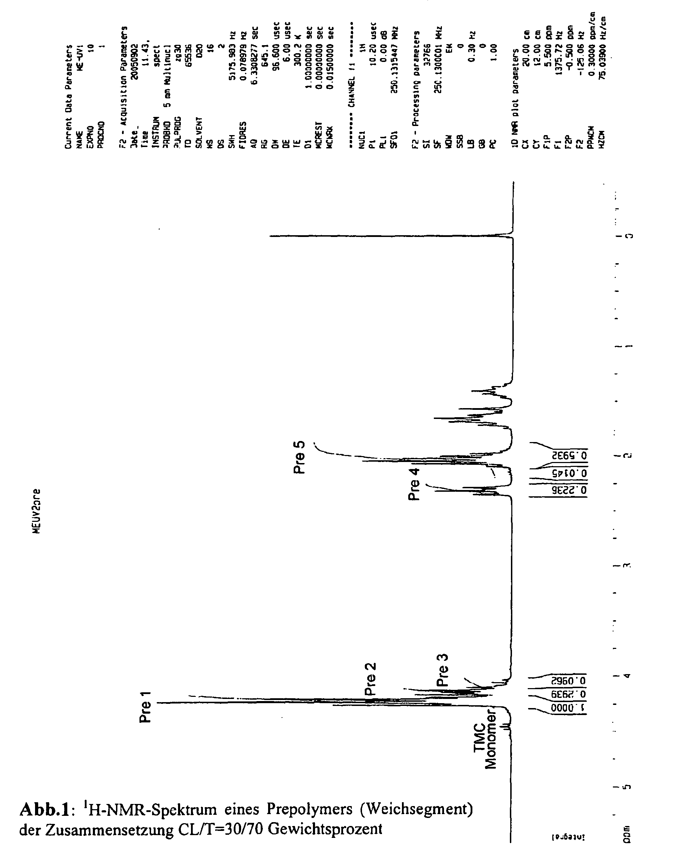

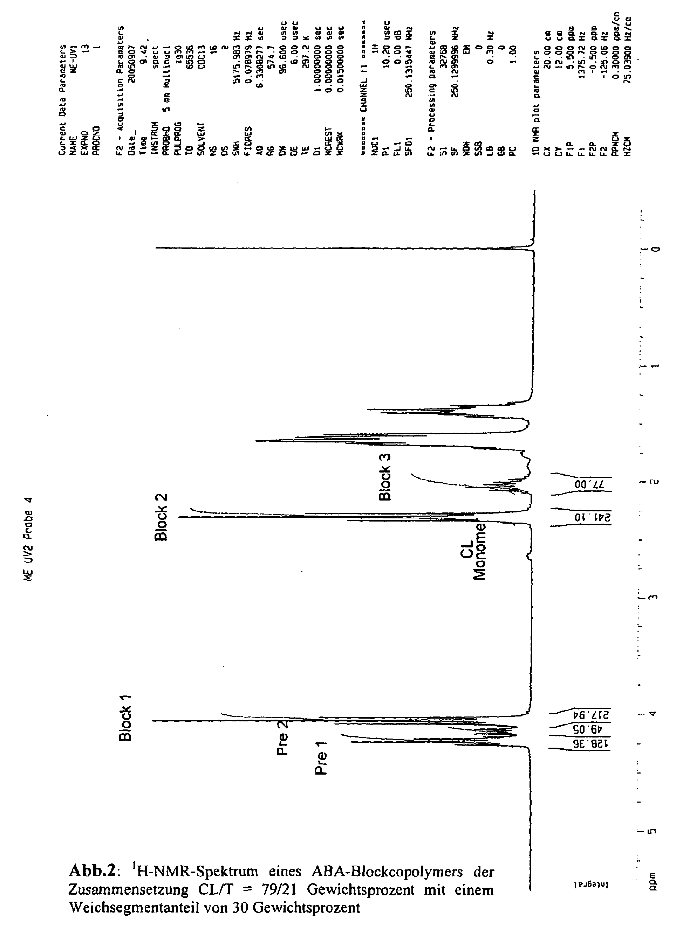

Zur Verfolgung der Polymerisation und einer möglichen Umesterung eignet sich in besonderer Weise die NMR Spektroskopie. Spektren des Prepolymers (isoliertes Weichsegment) und des Blockpolymers zeigen die Abbildungen 1 und 2. Die Zuordnungen der Peaks sind in der nachfolgenden Tabelle 1 aufgelistet. Das Prepolymer des Beispiels aus Abb. 1 besitzt die Zusammensetzung CL/T = 30/70, das daraus hergestellte Blockpolymer aus Abb. 2 die Zusammensetzung CL/T = 79/21. Der Weichsegmentanteil im Blockpolymer beträgt 31 Gew.-%.

Table 1: Zuordnung der NMR-Peaks der Abb. 1 und 2

| Peak | zugeodnet zu | Anzahl Protonen | Bemerkung |
|---|---|---|---|
| Pre1 | TMOm Weichsegment | 4 | Tellüberlagerung mit Pre2 |
| Pre2 | CL im Weichsegment, chemisch an TMC gebunden | 2 | Tellübenagerung mit Pre1 |
| Pre3 | CL im Weichsegment, chemisch an weiteres CL gebunden | | Teilübetlagerung mit Pre2 |
| Pre4 | CL im Weichsegment | 2 | dient zur Berechnung des CL-Antells im Prepolymer |
| Pre5 | TMC im Weichsegment | 2 | dient zur Berechnung des TMC-Anteils im Prepolymer |

(fortgesetzt)

| Peak | zugeodnet zu | Anzahl Protonen | Bemerkung |
|---|---|---|---|
| Block1 | CL Im Hartsegment (chemisch an weiteres CL gebunden) | 2 | Tellübenagerung mit Pre2 |
| Block2 | CL Gesamt im Blockpolymer | 2 | dient zur Berechnung des CL-Anteils im Blockpolymer |
| Block3 | TMC Im Blockpolymer | 2 | dient zur Berechnung des TMC-Anteils im Blockpolymer |
| TMC Monomer | Restmonomer TMC | 4 | dient zur Berechnung des TMC-Restmonomergehaltes |
| CL Monomer | Restmonomer CL | 2 | dient zur Berechnung des CL-Restmonomergehattes |

Die Integrale der Peaks Pre4 und Pre5 dienen zur Berechnung der chemischen Zusammensetzung des Weichsegments, die der Peaks Block 2 und Block 3 zur Berechnung der chemischen Zusammensetzung des Blockpolymers. Diese Peaks geben jedoch keine Auskunft darüber, ob während der Hartsegmentpolymerisation eine Umesterung stattgefunden hat und ob das Polymer mehrheitlich als statistisches Copolymer oder als Blockcopolymer vorliegt.

Eine Auskunft hierüber gibt das Verhältnis aus den Integralen der Peaks Pre1/Pre2/Pre3 in Verbindung mit Block1, allerdings nur empirischer Natur, da sich die Peaks in Teilen überlagern.

Peak Pre 1 stammt hauptsächlich vom Trimethylencarbonat des Weichsegmentes. Das Caprolacton des Weichsegmentes spaltet in 2 Teilpeaks zwischen 4,00 und 4,14 ppm auf, je nachdem, ob die betrachtete Caprolactoneinheit an weiteres Caprolacton (Peak Pre 3) oder an Trimethylencarbonat (Peak Pre 2) gebunden ist. Da im betrachteten Prepolymer mehr als die doppelte Menge an Trimethylencarbonat im Vergleich zu Caprolacton vorhanden ist und das Weichsegment ein statistisches Copolymer darstellt, ist eine Caprolacton-Einheit meist an Trimethylencarbonat gebunden (Peak Pre 2), nur ein geringerer Anteil der Caprolacton-Einheiten ist chemisch an eine weitere Caprolacton-Einheit gebunden (Peak Pre 3).

Im NMR Spektrum des Blockpolymers sieht man rechts neben Peak Pre2 den großen Peak des vom Hartsegment und vom Pre 3-Peak des Weichsegmentes stammenden Caprolactons: Im Hartsegment ist mit Ausnahme der Caprolacton-Einheiten, die direkt an den HydroxylEndgruppen des Prepolymers angeknüpft sind, idealerweise jede Caprolacton-Einheit mit einer weiteren Caprolacton-Einheit chemisch verknüpft.

Verläuft das Anpolymerisieren des Hartsegments ohne Umesterung und Insertion des Monomers in das Prepolymer, so bleibt das Verhältnis der Integrale aus Pre1 zu Pre2 vom Prepolymer- zum Blockpolymerspektrum nahezu unverändert, das Integral von Block 1 zu Block 2 gibt in etwa prozentual an, wie viel der Gesamtcaprolacton-Menge weiteres Caprolacton als Nachbar besitzt und somit weitestgehend (nach Abzug des Pre 3-Peaks) dem Hartsegment zugeordnet werden kann.

Findet dagegen eine deutliche Umesterung bzw. Insertion statt, ist das Verhältnis aus Pre1 zu Pre2 im Blockcopolymer im Vergleich zum Weichsegment erniedrigt und das Verhältnis aus Block1 zu Block 2 ist deutlich geringer, als es der Theorie entsprechend zu erwarten ist.

Zur rechnerischen Bestimmung bietet sich folgende Methode an: Da die absolute Menge an TMC im Block- (Peak Block 3) als auch im Prepolymer (Peak Pre 5) die gleiche ist, werden alle Integrale des Blockpolymers auf das Integral von Block 3 und alle Integrale des Prepolymers auf das Integral von Pre 5 normiert (Index n).

Aus der Formel

$$(\text{Block1}_n - \text{Pre 3}_n)/(\text{Block2}_n - \text{Pre 4}_n)*100 \text{ \%} = \text{CL-CL [\%]}$$

lässt sich der Anteil des zur Stufe 2 zugegebenen Caprolactons berechnen, der im Polymer an weiteres Caprolacton gebunden vorliegt (CL-CL) und somit das Hartsegment bildet. Je näher dieser Wert bei 100 % liegt, desto geringer ist der Umesterungs-bzw. Insertionsanteil während der Hartsegmentpolymerisation.

Dieser Anteil liegt bei den erfindungsgemäßen Polymeren bei > 88 % für Polymere mit Weichsegmentanteilen > 40 Gewichtsprozent und kann bis zu 98 % für geringe Weichsegmentanteile von 25 -30 Gewichtsprozent betragen.

Aus dem Genannten geht hervor, dass die Umesterungs- bzw. Insertionstendenz mit steigendem Weichsegmentanteil zunimmt und schwerer kontrollierbar wird. Dies erklärt sich daraus, dass dem in der zweiten Stufe zugegebenen Caprolacton bei hohen Weichsegmentanteilen viele Bindungen zur Insertion zur Verfügung stehen oder aber viele Caprolacton- und Trimethylencarbonateinheiten durch Umesterung in das gebildete Hartsegment eingebaut werden können.

Zwar lassen sich auch bei Hartsegmentpolymerisationstemperaturen von über 150 °C im kleinen Maßstab und unter glücklicher Wahl der Initiator- und Katalysatorkonzentration Polymere mit höherem Hartsegmentanteil bilden, die teilkristallin sind und CL-CL-Werte bis zu 90 % besitzen können, allerdings ist hierfür ein extrem genaues Abpassen des richtigen Zeitpunktes für die Beendigung der Polymerisation und ein sehr schnelles Austragen aus dem Reaktor notwendig. Die Schmelzpunkte der so hergestellten Polymere liegen in der Regel trotzdem 5 °C bis 10 °C tiefer als die der erfindungsgemäßen Polymeren, was von großem Nachteil ist, da für viele medizinische Applikationsformen ein Schmelzpunkt von mindestens 40 °C Voraussetzung ist. Auch die Schmelzenthalpien (und damit die Kristallinität dieser Polymere) liegen im Vergleich zu den erfindungsgemäßen Polymeren cirka 10 % niedriger. Die Reproduzierbarkeit der Polymerisationen bei höheren Temperaturen als 150 °C ist deutlich herabgesetzt.

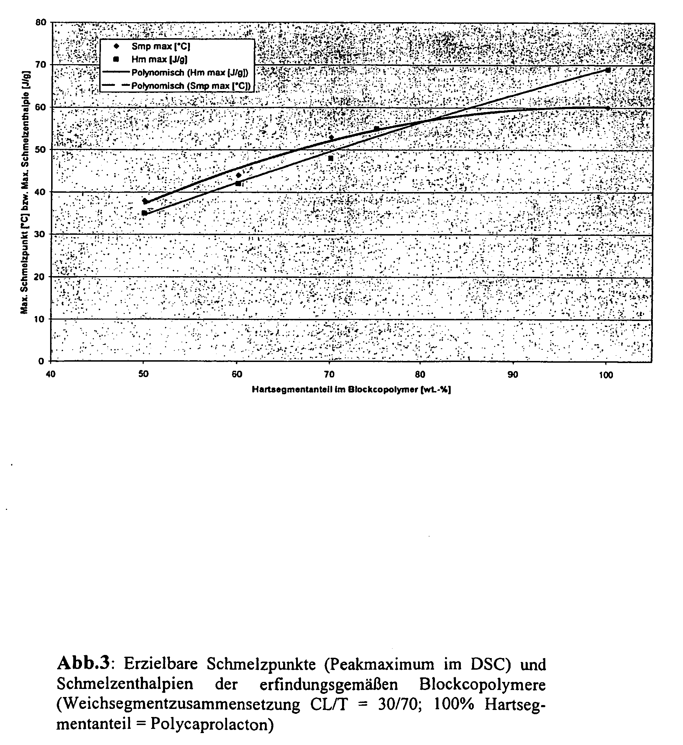

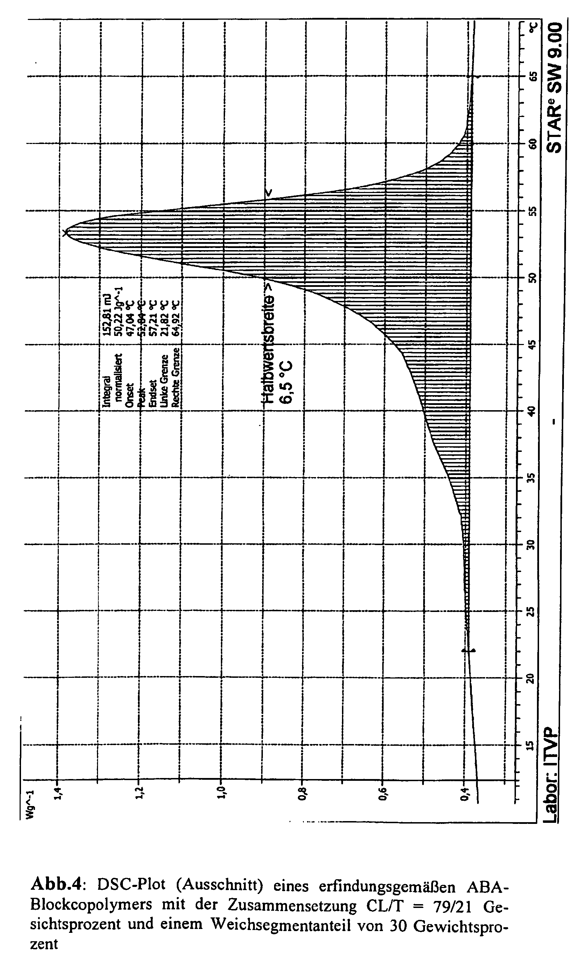

Abbildung 3 zeigt in Abhängigkeit vom Hartsegmentanteil der erfindungsgemäßen Triblockcopolymere deren maximal zu erzielende Schmelzpunkte und Schmelzenthalpien. Die DSC-Plots der erfindungsgemäßen Polymere zeigen im Vergleich zu den bei höheren Temperaturen hergestellten Polymeren einen engeren Schmelzbereich (vgl. Abb.4).

Die Molekulargewichtsregelung der erfindungsgemäßen Polymere erfolgt hauptsächlich durch Variation der Katalysator- und Initiatorkonzentration. Höhere Konzentrationen führen im ersten Polymerisationsschritt dazu, dass die Gesamtzahl der Polymerketten zunimmt und diese kürzerkettig sind. Die Zunahme der Kettenlänge relativ zum Prepolymer im zweiten Polymerisationsschritt wird hauptsächlich durch das Verhältnis von Weichsegment- zu Hartsegmentanteil bestimmt. Besonders bei höheren Hartsegmentpolymerisationstemperaturen, d.h. solchen über 150 °C, besteht aber zusätzlich die Gefahr, dass das zur zweiten Stufe zugegebene Caprolacton nicht ausgehend von den Hydroxylendgruppen des Weichsegments polymerisiert, sondern hohe Homopolymeranteile bildet, die dann mit den Weichsegmentketten nicht chemisch verknüpft sondern nur als Blend vorliegen.

Zusammenfassend gilt, dass die Tendenz zur Umesterung /Insertion während der Hartsegmentpolymerisation und die damit verbundene Abnahme des CL-CL-Wertes mit a) zunehmender Hartsegmentpolymerisationstemperatur und b) zunehmender Katalysator- und Initiatorkonzentration und c) zunehmendem Weichsegmentanteil zunimmt und dadurch die Schmelztemperatur und die Schmelzenthalpie der ABA-Blockcopolymere, gebildet aus Trimethylencarbonat und Caprolacton und gegebenenfalls Glykolid abnimmt und zu vollständig amorphen Polymeren führen kann. Die vorliegende Erfindung überwindet dieses Problem. Gemäß einer besonders bevorzugten Ausführungsform der Erfindung können Polymere erzeugt werden, deren Schmelztemperaturen und Schmelzenthalpien das erreichbare Maximum unter dem gegebenen Bereich der Zusammensetzung darstellen. Dies wird bei einer bevorzugten Ausführungsform der Erfindung dadurch erreicht, dass die Weichsegmentpolymerisation bis zu einem Umsatz von mindestens 98 % und eine Hartsegmentpolymerisationstemperatur (vor Zugabe des Caprolactons der zweiten Polymerisationsstufe) zwischen 130 und 150 °C gefahren wird, das Gesamtmonomer- zu Katalysatorverhältnis M/K zwischen 5.000 (mol/mol) und 120.000 (mol/mol) und bevorzugt im Bereich zwischen 10.000 (mol/mol) und 100.000 (mol/mol) und das Molverhältnis von Initiator zu Katalysator im Bereich zwischen 2 und 20 liegt.

Die erfindungsgemäßen Blockcopolymere eignen sich hervorragend zur Herstellung einer Vielzahl an Medizinprodukten, wobei diese ganz oder nur teilweise aus dem erfindungsgemäßen Polymer bestehen können. Ohne die Zahl der Anwendungen einzuschränken, seien hier genannt: Folien und Membranen zur Wundabdeckung und zur Adhäsionsprophylaxe, Beschichtungen für Medizinprodukte wie chirurgisches Nahtmaterial, das Covering sowie das Verkleben der Kreuzungspunkten von Stents, Kapillarmembranen und Hohlröhren zur Verwendung als Nervenleitschienen sowie zur Drainage, Trägermaterial für drug-delivery-Produkte, resorbierbares mono- und multifiles Nahtmaterial sowie Stents und temporäre Gefäßverschlüsse. Die thermoplastischen Eigenschaften der erfindungsgemäßen Blockcopolymere ermöglichen die Herstellung von Produkten aus der Schmelze, z.B. durch Spritzguß oder Extrudieren.

Nervenleitschienen, ganz oder teilweise gebildet aus dem erfindungsgemäßen Polymer, können sowohl als poröse Kapillarmembran wie auch als Schlauch bzw. Hohlröhre mit geschlossener Wandung vorliegen. Sie zeichnen sich durch hohe Flexibilität, durch Elastizität, durch ihr geringes Quellvermögen und durch die geringe pH-Wert-Absenkung im Verlauf der Degradation aus. Die in-vitro-Degradation in Sörensen Puffer bei 37 °C und pH 7,4 ist je nach Gesamtzusammensetzung und Weichsegmentanteil im wesentlichen zwischen 10 und 25 Wochen abgeschlossen.

Die Nervenleitschienen mit Membranstruktur in der Wandung lassen sich aus dem erfindungsgemäßen Polymer mittels zweier Methoden herstellen. Für beide Verfahren wird das erfindungsgemäße Polymer in einem halogenfreien Lösemittel wie Essigsäureethylester oder Dimethylacetamid gelöst. Der Polymerlösung kann ein Porenbildner wie beispielsweise Polyethylenglykol zugesetzt werden.

Beim Dip-coating-Prozeß wird ein Glas- oder Teflonstab in die Polymerlösung eingetaucht, unter Rotation herausgeführt und anschließend in ein Fällbad getaucht.

Das Fällmedium kann Wasser und bevorzugt eisgekühltes Wasser sein und zur besseren Ablösbarkeit der gebildeten Kapillarmembran vom Glas- oder Teflonstab mit Tensid versetzt sein. Es können auch andere Fällmedien verwendet werden, wobei gilt, dass das Lösemittel mit dem Fällmedium mischbar sein muss und die Fällgeschwindigkeit nicht zu gering ist.

In ähnlicher Weise kann eine solche Kapillarmembran auch kontinuierlich über ein Nassspinnverfahren hergestellt werden. Dabei wird die zuvor entgaste Polymerlösung, die zur Einstellung ihrer Viskosität auch beheizt werden kann, kontinuierlich durch eine Pumpe gefördert und über eine Zweistoffdüse ausgetragen. Im Kern der Zweistoffdüse wird Fällmittel über eine zweite Pumpe zudosiert. Das Spinngut wird durch ein Fällbad geführt und anschließend auf einer geeigneten Vorrichtung aufgespult. Die Größe des Lumens und die Wandstärke sind durch die Düsengeometrie, die pro Zeiteinheit geförderte Lösungsmenge und die Abzugsgeschwindigkeit einstellbar.

Die Porenstruktur des Dip-Coating-Prozesses unterscheidet sich vom kontinuierlichen Nassspinnen dadurch, dass sie stärker asymmetrisch ist und eine ausgeprägtere Skinschicht besitzt.

Ziel der Beschichtung von chirurgischen Nahtmaterialien, insbesondere von multifilem Nahtmaterial mit Geflechtsstruktur, ist die Erzielung einer Kombination aus gutem Fadenlauf, guter Nachplatzierbarkeit und hoher Knotensicherheit (Knotenhalt). Die Beschichtung soll daher zum einen dazu dienen, die raue Geflechtoberfläche zu glätten, das Gleitvermögen und damit die Gleitreibung zu vermindern und zum anderen bei einem gebildeten Knoten das Aufgehen der Schlinge zu verhindern. Letzteres bedingt, dass die Beschichtung entweder durch Bildung eines Berges beim Knüpfen ein mechanisches Hindernis darstellt oder zu einer gegenseitigen Verklebung führt oder durch elastischen Druck auf die Schlinge und wegen der durch die Elastizität ausgebildete Kuhle sowohl mechanisch als auch durch Haftreibung einen Widerstand darstellt . Letztgenannter Mechanismus kann in exzellenter Weise durch eine Beschichtung von chirurgischem Nahtmaterial mit dem erfindungsgemäßen Polymer realisiert werden. Im Gegensatz zum Stand der Technik, wo zur Erzielung eines befriedigenden Fadenlaufs und einer für den Chirurgen ausreichend guten Nachplatzierbarkeit zusätzlich Gleitmittel wie Erdalkalimetallsalze der Stearinsäure oder verwandte Verbindungen zur Beschichtung erforderlich sind, kann bei einer Beschichtung, hauptsächlich gebildet aus dem erfindungsgemäßen Polymer, auf diese Zusätze verzichtet werden, auch wenn sie bei manchen Anwendungen erwünscht sein können.

Die Beschichtung von chirurgischem Nahtmaterial mit dem erfindungsgemäßen Polymer kann auf sehr einfache und kostengünstige Art durchgeführt werden, in dem eine Lösung des Polymeren in halogenfreien organischen Lösemitteln wie Estern, Ketonen, Aldehyden oder Alkoholen im Konzentrationsbereich zwischen einem und zwanzig Gewichtsprozent und bevorzugt zwischen zwei und zehn Gewichtsprozent hergestellt und das zu beschichtende Nahtmaterial durch diese Lösung hindurchgezogen wird. Um das Abdampfen des Lösemittels und die Verfestigung der Beschichtung zu beschleunigen, wird der beschichtete Faden durch einen Heißluftkanal mit Temperaturen zwischen 60 °C und 190 °C und bevorzugt zwischen 80 °C und 160 °C geführt und anschließend mit Trockendruckluft zur Abkühlung vor dem Aufwickelvorgang angeblasen. Der Anteil der Beschichtung am chirurgischen Nahtmaterial wird über die Konzentration und die Viskosität der Beschichtungslösung so eingestellt, dass er zwischen einem und fünfzehn Gewichtsprozent und bevorzugt zwischen zwei und acht Gewichtsprozent beträgt.

## Beispiele

Wo nicht explizit anders beschrieben, wurden die nachfolgenden Charakterisierungsmethoden angewendet.

$^1$H-NMR-Spektren wurden auf einem Bruker-Spektrometer DPX 250 MHz aufgenommen und mittels der Software xwinnmr der Fa. Bruker ausgewertet.

Die Bestimmung der inhärenten Viskosität (i.V.) erfolgte bei einer Temperatur von 30 °C in Hexafluorisopropanol (HFIP) bei einer Konzentration von 0,5 Gewichtsprozent unter Verwendung eines Ubbelohde Viskosimeters 0a.

Die thermischen Eigenschaften (Glasübergangstemperatur, Schmelztemperatur und Schmelzenthalpie) wurden auf dem Modul DSC 30 mit einem TC 15 Controller (beide Fa. Mettler-Toledo) ermittelt. Hierzu wurden 3 bis 10 mg Probensubstanz in einem 40 $\mu$l-Aluminiumtiegel mit einer Heizrate von 10 °C/min gefahren. Zwischen dem ersten und zweiten run des DSC wurde die Probe mit maximaler Kühlrate (ca. 50 °C/min) abgekühlt. Die Auswertung der Plots erfolgte mit der STARe-Software der Fa. Mettler-Toledo.

In vitro-Degradationstests wurden in Sörensen-Lösung (pH 7,4) bei 37 °C durchgeführt. Die Reißkrafttests fanden auf einer Zwick-Zugprüfmaschine statt.

## Knotenhaltmessung mittels Streichtestmethode

Das zu prüfende Nahtmaterial wurde um zwei halbkreisförmige Backen geführt und mittels einem chirurgischen Knoten fixiert. Die Backen drückten dabei mit einer geringen Vorspannkraft nach außen. In einem zyklischen Verfahren wurde der Knoten in wässrigem Medium bis zu maximal 1000 mal von einer Silikonkautschuk-Lippe überstrichen. Die Anzahl der Zyklen bis zum Aufgehen des Knotens (oder bis zum Erreichen des 1000. Zyklus) wurden automatisch gezählt.

Knotenhaltmessung mittels Pulsationstest

Wie beim Streichtest wurde um die zwei halbkreisförmigen Backen, die unter einer Vorkraft von 1,3 N standen, das zu prüfende Nahtmaterial geknotet. Im Rahmen der zyklischen Prüfung wurde die nach außen drückende Kraft bis zu maximal 1000 mal zwischen 2,6 N und 3,2 N variiert. Die Anzahl der Zyklen bis zum Aufgehen des Knotens (oder bis zum Erreichen des 1000. Zyklus) wurden automatisch gezählt.

**Beispiel 1:** ABA-Triblockcopolymer gemäß Erfindung mit einer Gesamtzusammensetzung von CL/T = 79/21 Gew.-% und 30 Gew.-% Weichsegmentanteil der Zusammensetzung CL/T = 30/70 Gew.-%

In einem konischen 5 I-Reaktor mit Wendelrührer, Vakuum- und Schutzgasanschluß wurden für die erste Polymerisationsstufe 270 g Caprolacton und 630 g Trimethylencarbonat zusammen mit 1,0 g Diethylenglykol (bifunktioneller Initiator) und 0,2 g 2-Ethylhexansäure-Zinn (II)-Salz (Zinnoctoat als Katalysator) vorgelegt und über einen Zeitraum von 2 Stunden unter Anlegen von Vakuum getrocknet. Anschließend wurde unter Rühren die Reaktionstemperatur auf 210 °C erhöht und für 5 Stunden gehalten. Unter Schutzgasatmosphäre wurde eine Probe des Prepolymers (P1-pre) entnommen und charakterisiert.
Die Solltemperatur des Reaktors wurde daraufhin auf 145 °C abgesenkt. Nach Erreichen dieser Temperatur wurden 2.100 g Caprolacton zur Anpolymerisation der Hartsegmentblöcke (A) an das dihydroxyterminierte Weichsegment zugegeben.
Der Polymerisationsumsatz wurde anhand mehrerer NMR-Proben untersucht. Nach 5 Tagen Polymerisationsdauer bei 145 °C war der Monomergehalt an Caprolacton unter 2 Gew.-%, woraufhin das Polymer abgelassen wurde. Das im heißen Zustand noch transparente, geschmolzene Polymer wurde beim Abkühlen aufgrund eintretender Kristallisation weiß und opak. Von dem erstarrten Polymer wurde eine Probe entnommen (P1).
Die Eigenschaften des Pre- und Blockcopolymeren gibt Tabelle 2 wider.

**Beispiel 2:** ABA-Triblockcopolymer gemäß Erfindung mit einer Gesamtzusammensetzung von CL/T = 89,5/10,5 Gew.-% und 35 Gew.-% Weichsegmentanteil der Zusammensetzung CUT = 70/30 Gew.-%.

Durchführung analog zu Beispiel 1, jedoch mit 630 g Caprolacton und 270 g Trimethylencarbonat in Stufe 1 der Polymerisation und 1670 g Caprolacton in Stufe 2 der Polymerisation. Nach 4 Tagen und 15 Stunden Polymerisationsdauer bei 145 °C wurde der Monomergehalt an Caprolacton mittels NMR zu 1,3 Gew.-% bestimmt und das Polymer aus dem Reaktor abgelassen.
Die Eigenschaften des Pre- und Blockcopolymeren gibt Tabelle 2 wider.

**Beispiel 3:** Erfindungsgemäßes Polymer entsprechend Beispiel 1, jedoch mit geringerem Molekulargewicht.

Durchführung entsprechend Beispiel 1, jedoch unter Erhöhung der Katalysatoreinwaage von 0,2 g auf 0,9 g und der Initiatoreinwaage von 1,0 g auf 4,5 g. 65 Stunden nach Zugabe des Caprolactons zur Ausbildung des Hartsegmentes wurde eine NMR-Probe zur Bestimmung des Umsatzes entnommen, die zeigte, dass die Polymerisation abgeschlossen ist. Der Ablass des Polymers erfolgte nach 70 Stunden Hartsegmentpolymerisationsdauer.
Die Eigenschaften des Pre- und Blockcopolymeren gibt Tabelle 2 wider.

**Beispiel 4:** Versuch zur Herstellung eines niedermolekularen ABA-Triblockcopolymers mit einer Gesamtzusammensetzung von CL/T = 72/28 Gew.-% und 40 Gew.-% Weichsegmentanteil der Zusammensetzung CL/T = 30/70 Gew.-%.

In einem konischen 5 I-Reaktor mit Wendelrührer, Vakuum- und Schutzgasanschluß wurden für die erste Polymerisationsstufe 360 g Caprolacton und 840 g Trimethylencarbonat zusammen mit 6,8 g Diethylenglykol (bifunktioneller Initiator) und 1,4 g 2-Ethylhexansäure-Zinn (II)-Salz (Zinnoctoat als Katalysator) vorgelegt und über einen Zeitraum von 2 Stunden unter Anlegen von Vakuum getrocknet. Anschließend wurde unter Rühren die Reaktionstemperatur auf 210 °C erhöht und für 4,5 Stunden gehalten. Unter Schutzgasatmosphäre wurde eine Probe des Prepolymers (P4-pre) entnommen und charakterisiert.
Die Solltemperatur des Reaktors wurde daraufhin auf 145 °C abgesenkt. Nach Erreichen dieser Temperatur wurden 1800 g Caprolacton zur Anpolymerisation der Hartsegmentblöcke (A) an das dihydroxyterminierte Weichsegment zugegeben.
Nach 21 Stunden Hartsegmentpolymerisationsdauer wurde eine erste Probe (P4-1) entnommen, jedoch erst zusammen mit der zweiten Probe (P4-2), die nach 69 Stunden entnommen wurde, charakterisiert. Die NMR-Spektren zeigten, dass die Polymerisation nach 21 Stunden schon abgeschlossen war und das Polymer in der Folgezeit aufgrund Umesterung/Insertion stark randomisierte. Das abgelassene Polymer kristallisierte nach Abkühlung nicht aus, sondern blieb auch nach Lagerung bei Raumtemperatur vollständig amorph. Dies zeigt, dass die optimale Hartsegmentpolymerisationsdauer (bei gegebener Polymerisationstemperatur von kleiner gleich 150 °C) der erfindungsgemäßen Polymere speziell von den Faktoren Initiator- und Katalysatorkonzentration und Weichsegmentanteil abhängt.

Die Eigenschaften des Pre- und Blockcopolymeren gibt Tabelle 2 wider.

**Beispiel 5:** Nachstellung von Beispiel 4 als erfindungsgemäßes ABA-Triblockcopolymer mit hohem Blockcopolymercharakter und verringertem Molekulargewicht.

Weichsegmentpolymerisation analog zu Beispiel 4. Anschließend Absenken der Reaktionstemperatur auf 150 °C. Erste Probennahme (P5a) nach 15 Stunden, der Ablass des Polymer erfolgte 18 Stunden nach Hartsegmentpolymerisationsdauer (Probe P5b)

Die Eigenschaften des Pre- und Blockcopolymeren gibt Tabelle 2 wider.

**Beispiel 6:** ABA-Triblockcopolymer gemäß Erfindung mit einer Gesamtzusammensetzung von CUT = 85/15 Gew.-% und 30 Gew.-% Weichsegmentanteil der Zusammensetzung CL/T = 50/50 Gew.-%.

Durchführung analog zu Beispiel 2, jedoch mit 450 g Caprolacton und 450 g Trimethylencarbonat in Stufe 1 der Polymerisation und 2100 g Caprolacton in Stufe 2 der Polymerisation.

**Beispiel 7:** Vergleichsbeispiel eines nicht erfindungsgemäßen ABA-Triblockcopolymer mit einer Gesamtzusammensetzung von CL/T = 85/15 Gew.-% und 30 Gew.-% Weichsegmentanteil der Zusammensetzung CUT = 50/50 Gew.-%.

Einwaagen und Durchführung der Weichsegmentpolymerisation analog zu Beispiel 6. Für die Hartsegmentpolymerisation wurde die Reaktionstemperatur bei 210 °C belassen. Mit dem Ablass des Polymeren aus dem Reaktor wurde schon nach 90 Minuten begonnen. Das vollständige Ablassen dauerte ca. 15 Minuten.

Das abgelassene Polymer (Probe P7) benötigt im Vergleich zu Beispiel 6 länger zur Kristallisation und besitzt im Gegensatz zu diesem grießige Bestandteile, die besonders gegen Ablassende auftreten. Das Polymer ist nicht vollständig homogen, mit zunehmender Ablassdauer wird es gelbstichig. Die Geruchsbelästigung durch Restmonomer während des Austragens des Polymeren aus dem Reaktor ist hoch, es musste eine Absaugvorrichtung angebracht werden.

Die Eigenschaften des Polymeren gibt Tabelle 2 wider.

**Beispiel 8:** ABA-Triblockcopolymer gemäß Erfindung mit einer Gesamtzusammensetzung von CL/T = 65/35 Gewichtsprozent und 50 Gewichtsprozent Weichsegmentanteil der Zusammensetzung CL/T = 30/70 Gew.-%.

450 g Caprolacton werden zusammen mit 1050 g Trimethylencarbonat, 1,0 g Diethylenglykol (bifunktioneller Initiator) und 0,2 g 2-Ethylhexansäure-Zinn (II)-Salz (Zinnoctoat als Katalysator) vorgelegt. Die Durchführung der beiden Polymerisationsstufen erfolgt entsprechend Beispiel 1, aber mit 1500 g Caprolacton zur Stufe 2 und aufgrund des hohen Weichsegmentanteils mit einer verkürzten Hartsegmentpolymerisationsdauer von 82 Stunden.

Das abgelassene Polymer (Probe P8) erstarrt nach Abkühlung aufgrund eintretender Kristallisation und wird weiß und opak.

Die Eigenschaften des Pre- und Blockcopolymeren gibt Tabelle 2 wider.

**Beispiel 9:** ABA-Triblockterpolymer gemäß Erfindung mit einer Gesamtzusammensetzung von CUT/G = 79/15/6 Gewichtsprozent und 30 Gewichtsprozent Weichsegmentanteil der Zusammensetzung CL/T/G = 30/50/20 Gew.-%.

In einem konischen 5 I-Reaktor mit Wendelrührer, Vakuum- und Schutzgasanschluß wurden für die erste Polymerisationsstufe 270 g Caprolacton, 450 g Trimethylencarbonat und 180 g Glykolid zusammen mit 1,0 g Diethylenglykol (bifunktioneller Initiator) und 0,2 g 2-Ethylhexansäure-Zinn (II)-Salz (Zinnoctoat als Katalysator) vorgelegt und über einen Zeitraum von 2 Stunden unter Anlegen von Vakuum getrocknet. Anschließend wurde unter Rühren die Reaktionstemperatur auf 210 °C erhöht und für 5 Stunden gehalten. Unter Schutzgasatmosphäre wurde eine Probe des Prepolymers (P9-pre) entnommen und charakterisiert.

Die Solltemperatur des Reaktors wurde daraufhin auf 145 °C abgesenkt. Nach Erreichen dieser Temperatur wurden 2.100 g Caprolacton zur Anpolymerisation der Hartsegmentblöcke (A) an das dihydroxyterminierte Weichsegment zugegeben.

Der Polymerisationsumsatz wurde anhand mehrerer NMR-Proben untersucht. Nach 23 Stunden Polymerisationsdauer bei 145 °C war der Monomergehalt an Caprolacton unter 2 Gew.-%, woraufhin das Polymer abgelassen wurde. Das im heißen Zustand noch transparente, geschmolzene Polymer wurde beim Abkühlen aufgrund eintretender Kristallisation ockerfarben und opak. Von dem erstarrten Polymer wurde eine Probe entnommen (P9).

Die Eigenschaften des Pre- und Blockcopolymeren gibt Tabelle 2 wider.

**Beispiel 10:** Sternförmiges, dreiästiges ABA-Blockcopolymer gemäß Erfindung mit einer Gesamtzusammensetzung von CL/T = 79/21 Gew.-% und 30 Gew.-% Weichsegmentanteil der Zusammensetzung CL/T = 30/70 Gew.-%, initiiert mit Glycerin.

Durchführung wie Beispiel 1, jedoch statt 1,0 g Diethylenglykol Verwendung von 0,67 g Glycerin als Initiator. Die Reaktionsdauer in Stufe 2 der Polymerisation betrug 68 Stunden.

Die Eigenschaften des Pre- und Blockcopolymeren gibt Tabelle 2 wider.

| Beispiel | Typ laut Ansatz | erfindungsgemäß | Overall CL/T/G [wt.-%] | WS CL/T/G [wt.-%] | WS-Anteil [wt.-%] | HS-Anteil [wt.-%] | CL-CL [%] | TgWS[°C] | TgABA [°C] | Hm[J/g] | Smp[°C] | i.V. [dl/g] |
|---|---|---|---|---|---|---|---|---|---|---|---|---|
| 1 | ABA | ja | 79/21/0 | 30170/0 | 30 | 70 | 97.2 | -37 | -54 | 47 | 52 | 1,57 |
| 2 | ABA | ja | 89.5110,5/0 | 70/3010 | 35 | 65 | 95,8 | -51 | -56 | 45 | 49 | 1,48 8 |
| 3 | ABA | ja | 79/21/0 | 30/70/0 | 30 | 70 | 97.8 | -38 | -51 | 50 | 52 | 1,08 |
| 4 | ABA | nein | 72/28/0 | 30/7010 | 40 | 60 | 75,0 | -37 | -57 | 0 | n.b. | 0,99 |
| 5 | ABA | ja | 72/28/0 | 30/70/0 | 40 | 60 | 92.3 | -37 | -57 | 41 | 44 | 1,08 |
| 6 | ABA | ja | 85/15/0 | 50/50/0 | 30 | 70 | 96,9 | -45 | -55 | 48 | 51 | 1,39 9 |
| 7 | ABA | nein | 85/15/0 | 50/50/0 | 30 | 70 | 86,3 | -45 | -57 | 30 | 44 | 1,27 |
| 8 | ABA | ja | 6513510 | 30/7010 | 50 | 50 | 92.6 | -38 | -45 | 34 | 39 | 1,45 |
| 9 | ABA | ja | 79/15/6 | 30150/20 | 30 | 70 | 94,3 | -33 | -49 | 47 | 51 | 1,38 |
| 10 | ABA Stern | ja | 79/21/0 | 30170/0 | 30 | 70 | 97,9 | -37 | -58 | 45 | 49 | 1,13 |

WS-Anteil:      Weichsegmentanteil am Gesamtpolymeren
HS-Anteil:      Hartsegmentanteil am Gesamtpolymeren
Tg WS:      Glasumwandlungspunkt einer isolierten Probe des Weichsegmentes
Tg ABA:      Glasumwandlungspunkt des fertigen Polymeren
Hm:      Schmelzenthalpie (heat of fusion)
Smp:      Schmelzpunkt (Maximum des Schmelzpeaks im DSC)
i.V.:      inhärente Viskosität

**Beispiel 11:** Herstellung einer Kapillarmembran mittels Dip-Coating-Prozeß zum Einsatz als Nervenleitröhre:

15 g des Polymeren aus Beispiel 1 wurden zusammen mit 10 g PEG (MG = 20.000) als Porenbildner bei 60 °C in 75 g Dimethylacetamid (DMAC) gelöst. In die Polymerlösung wurde mehrmals ein Stab mit 1 mm Durchmesser eingetaucht und unter Drehen wieder herausgeführt. Anschließend wurde das am Stab anhaftende Polymer im Wasserbad gefällt und mehrmals mit Wasser nachgewaschen. Die vom Stab abgelöste Kapillarmembran besaß eine innere Skinschicht, einen Außendurchmesser von 1,3 mm und eine Wandstärke von 150 $\mu$m.

**Beispiel 12.** Herstellung einer Kapillarmembran durch kontinuierliches Nassspinnen und Fällen in Eiswasser.

Für 1 kg einer Spinnlösung wurden 200 g des Polymeren aus Beispiel 1 und 150 g PEG (MG = 20.000 g/mol) als Porenbildner in 650 g N-Methylpyrrolidon (NMP) unter Rühren bei 80 °C gelöst und anschließend noch heiß filtriert. Die filtrierte Lösung wurde nachfolgend im Vakuum entgast. Die so vorbereitete Polymerlösung wurde über eine Zweistoffspinndüse (Außendurchmesser 1mm, Innendurchmesser 0,45 mm) zur Kapillaren versponnen, indem durch den inneren Düsenring als Fällmittel Wasser zudosiert wurde. Als äußeres Fällmittel dient ein Eiswasserbad. Die Endloskapillare wurde in cirka 10 cm lange Stücke geschnitten und über Nacht gründlich mit Wasser gespült. Die so ersponnenen Kapillaren besitzen einen Außendurchmesser von cirka 900 $\mu$m und eine Wandstärke von 150 $\mu$m. Die Wand ist als asymmetrische Membran ausgebildet.

Nach Degradation in Sörensenpuffer bei pH 7,4 und einer Temperatur von 37 °C besaßen die porösen Membranen nach 8 Wochen noch ungefähr 50 % ihrer Ausgangsreißkraft. Nach $\gamma$-Sterilisation mit einer Strahlendosis von 10 kGy wurde überraschend eine Verlangsamung der Degradationsgeschwindigkeit festgestellt.

Bei den Beispielen 13 bis 15 wurde auf einem Doppelschneckenextruder des Typs TW100 in Verbindung mit einem Haake-Rheocord90-System extrudiert.

**Beispiel 13:** Herstellung eines Monofilaments aus dem Polymer von Beispiel 1:

Das zuvor über Nacht im Vakuum getrocknete Polymer aus Beispiel 1 wurde unter Schutzgasatmosphäre der Einzugszone des Extruders zugeführt. Die Düsentemperatur des Extruders betrug 110 °C, eingebaut war eine 1,25 mm Einlochdüse mit einem UD-Verhältnis von 4. Nach Verlassen der Düse durchlief das Extrudat einen Airgap von 2 cm und wurde anschließend in einem Wasserbad mit 20 °C zur Verfestigung abgekühlt, mittels eines Galettenduos abgezogen und auf Spulen gewickelt. Bei einer Schneckendrehzahl von 4 U/min und einer Abzugsgeschwindigkeit von 4,5 m/min ergab sich ein Spinnfadendurchmesser von 0,7 mm. Das unverstreckte Extrudat konnte von Hand bei Raumtemperatur um 800 Prozent gelängt werden.

Zur Erhöhung der Festigkeit und Verringerung der Reißdehnung wurde der Spinnfaden kontinuierlich zwischen zwei Galettenduos bei Raumtemperatur verstreckt, indem die Geschwindigkeit der zweiten Galette 8 mal höher gewählt wurde als die der Liefergalette. Der Durchmesser des Monofilaments veringerte sich durch die Verstreckung auf 0,27 mm. Die mechanischen Kenndaten und die Ergebnisse aus dem Degradationstest sind in Tabelle 3 aufgelistet.

**Beispiel 14:** Monofilament aus Polymer von Beispiel 9 (enthält 6 % Glykolid)

Das Polymer aus Beispiel 9 wurde analog zu Beispiel 13 zu einem Monofilament verarbeitet. Der Durchmesser des Spinnstranges betrug 0,6 mm. Das Verstreckverhältnis lag mit 7,4 etwas niedriger als das aus Beispiel 13, der Durchmesser des verstreckten Monofilaments betrug 0,21 mm.

Die mechanischen Kenndaten und die Ergebnisse aus dem Degradationstest sind in Tabelle 3 aufgelistet. Der Biegemodul ist mit 330 N/mm$^2$ nur cirka halb so groß wie der von monofilem Standardnahtmaterial (Monocryl), woraus eine hervorragende Flexibilität resultiert.

Tabelle 3: Kenndaten Monofilamente

| Beispiel | DM [mm] | LRK [N] | LRK [N/mm$^2$] | Elongation [%] | KRK-[N] | KRK [N/mm$^2$] | Retention 12 Wochen [%] | Retention 20 Wochen [%] |
|---|---|---|---|---|---|---|---|---|
| 13 | 0,27 | 14,3 | 250 | 74 | 13,0 | 227 | 85 | 78 |

(fortgesetzt)

| Beispiel | DM [mm] | LRK [N] | LRK [N/mm$^2$] | Elongation [%] | KRK-[N] | KRK [N/mm$^2$] | Retention 12 Wochen [%] | Retention 20 Wochen [%] |
|---|---|---|---|---|---|---|---|---|
| 14 | 0,21 | 7,9 | 228 | 125 | 8,1 | 234 | 54 | 31 |

| | |
|---|---|
| DM: | Durchmesser |
| LRK: | Linearreißkraft |
| Elongation: | Bruchdehnung |
| KRK: | Knotenreißkraft |
| Retention x Wochen: | Linearreißkraftretention nach x Wochen Lagerung in Sörensen-Puffer bei T = 37 °C |

Es ist zu erwarten, dass nach einer Optimierung der Extrusions-, Verstreck- und Temperungsparameter die Festigkeit der Monofilamente soweit erhöht werden kann, dass alle Stärken die Anforderungen der USP und EP erfüllen. Dann steht ein extrem flexibles, langzeitresorbierbares Nahtmaterial zur Verfügung, das bei der Degradation zudem extrem wenig Säure freisetzt. Aus den Monofilamenten können durch den weiteren Einsatz bekannter Technologien Netze, Gelege, Gestricke und Röhrengeflechte (Stents) gefertigt werden.

**Beispiel 15**: Extrusion eines Schlauches mittels Katheterdüse aus den Polymeren aus Beispiel 1

Am TW100-Doppelschneckenextruder wurde eine horizontale Katheterdüse mit Außendurchmesser 4,0 mm und Innendurchmesser 2,0 mm angeflanscht. Als Stützgas wurde Stickstoff mit konstantem Fluß durch die Kernbohrung der Düse geleitet. Unmittelbar nach der Düse wurde ein auf 10 °C gekühltes Wasserbad platziert, dessen Wasserspiegel nur ca. 1 cm tiefer lag als die Düsenkapillare. Hinter dem Bad wurde ein spezieller Wickler genutzt, um den dann verfestigten Schlauch mit definierter Geschwindigkeit aufzuwickeln. Die Düsentemperatur wurde auf 70 °C gesetzt, die Schneckendrehzahl betrug 6 U/min. Für Versuch 15 a betrug die Abzugsgeschwindigkeit am Wickler 1,3 m/min, bei Versuch 15 b 0,65 m/min. Für die Versuche 15 a und 15 b betrug der Stützgasfluß 8 Skalenteile. Für Versuch 15 c wurde dieser auf 20 Skalenteile erhöht, abgezogen wurde bei einer Geschwindigkeit von 1,6 m/min. Der Düsendruck lag bei allen Versuchen bei 27 bar.

Die erhaltenen Schläuche zeigten eine sehr hohe Flexibilität. Ein Teil der extrudierten Schläuche wurde zusätzlich im Schlitzheizer bei T = 50 °C und einem Verstreckverhältnis von 1: 6,7 verstreckt.

Tabelle 4 gibt einen Überblick über die erhaltenen Dimensionen der Schläuche.

Die extrudierten Schläuche (verstreckt und unverstreckt) mit nicht poröser Wandstruktur eigenen sich in hervorragender Weise als Nervenleitschienen für kurze Defektstrecken bis cirka 25 mm und als Drainageschläuche wie beispielsweise im Hirn- oder ophthalmologischen Bereich.

Tabelle 4: Schlauchdimensionen

| Beispiel | ADM[mm] | Wann [mm] REM | IDM [mm] REM |
|---|---|---|---|
| 15 a | 1,800 | 0,350 | 1,100 |
| 15 b | 2,600 | 0,300 | 2,000 |
| 15 c | 2,100 | 0,150 | 1,800 |
| 15 a verstreckt | 0,900 | 0,100 | 0,700 |
| 15 b verstreckt | 1,100 | 0,080 | 0,940 |
| 15 c verstreckt | 1,350 | 0,050 | 1,250 |

| | |
|---|---|
| ADM: | Außendurchmesser |
| IDM: | Innendurchmesser |
| Wand: | Wandstärke |

**Beispiel 16**: Beschichtung von multifilem chirurgischem Nahtmaterial mit dem erfindungsgemäßen Polymer aus Beispiel 3 aus 4,5 % Lösung.

45 g des Polymers aus Beispiel 3 wurden in 955 g Ethylacetat gelöst. Durch diese Lösung wurde ein Faden der Stärke USP 2/0 mit einer konstanten Geschwindigkeit von 20 m/min kurz durchgeführt und beschichtet. Nach Verlassen des Beschichtungsbades wurde der Faden bei ca. 80°C im Heizkanal getrocknet und aufgespult. Der Beschichtungsgehalt wurde mittels NMR zu 1,72 Gew.% bestimmt.

Der Rutschknoten SxSxS hielt im Streichtest im Durchschnitt beim Industriestandard 378 Zyklen (Mittelwert aus 24 Tests), bevor der Knoten aufging, beim mit dem Triblockcopolymer beschichteten Faden im Durchschnitt 689 Zyklen (Mittelwert aus 18 Tests)

Der Rutschknoten SxSxS hielt im Pulsationstest im Durchschnitt beim Industriestandard 575 Zyklen, bevor der Knoten aufging, beim mit dem Triblockcopolymer beschichteten Faden im Durchschnitt 608 Zyklen (Mittelwert aus 12 Tests)

Der mit dem Triblockcopolymer beschichtete Faden ist beim SxSxS (Rutschknoten) sicherer als der Standardfaden.

**Beispiel 17**: Beschichtung von multifilem chirurgischem Nahtmaterial mit dem erfindungsgemäßen Polymer aus Beispiel 3 aus 6,0 % Lösung.

60 g des Polymers aus Beispiel 3 wurden in 940 g Ethylacetat gelöst. Durch diese Lösung wurde ein Faden der Stärke USP 2/0 mit einer konstanten Geschwindigkeit von 20 m/min kurz durchgeführt und beschichtet. Nach Verlassen des Beschichtungsbades wird der Faden bei ca. 80°C im Heizkanal getrocknet und aufgespult. Der Beschichtungsgehalt wurde mittels NMR zu 2,46 Gew.% bestimmt.

Der Rutschknoten SxSxS hielt im Streichtest im Durchschnitt beim Industriestandard 378 Zyklen (Mittelwert aus 24 Tests), bevor der Knoten aufging, beim mit dem Triblockcopolymer beschichteten Faden im Durchschnitt 632 Zyklen (Mittelwert aus 18 Tests)

Der Rutschknoten SxSxS hielt im Pulsationstest im Durchschnitt beim Industriestandard 575 Zyklen (Mittelwert aus 24 Tests), bevor der Knoten aufging, beim mit dem Triblockcopolymer beschichteten Faden im Durchschnitt 889 Zyklen (Mittelwert aus 18 Tests)

Der mit dem Triblockcopolymer beschichtete Faden ist beim SxSxS (Rutschknoten) sicherer als der Standardfaden.

**Beispiel 18**: Beschichtung von multifilem chirurgischem Nahtmaterial mit dem erfindungsgemäßen Polymer aus Beispiel 3 (5,0 % Lösung) unter Zugabe von 5 % Calciumstearat

50 g des Polymeren aus Beispiel 3 und 50 g Calciumstearat wurden in 900 g Xylol bei 140°C gelöst. Anschließend wurde die erkaltete Lösung mit einem starken Rührer zu einem dünnflüssigen Beschichtungsbad homogenisiert. Durch dieses Beschichtungsbad wurde bei einer Temperatur von 22°C ein Faden der Stärke USP 3/0 mit einer konstanten Geschwindigkeit von 20 m/min kurz eingetaucht und beschichtet. Nach Verlassen des Beschichtungsbades wurde der Faden bei 160°C im Heizkanal getrocknet und aufgespult. Der Beschichtungsgehalt wurde mittels NMR zu 4,71Gew.% bestimmt.

Der Rutschknoten SxSxS hielt im Streichtest im Durchschnitt beim Industriestandard 525 Zyklen (Mittelwert aus 12 Tests), bevor der Knoten aufging, beim mit dem Triblockcopolymer beschichteten Faden im Durchschnitt 1000 Zyklen (Mittelwert aus 6 Tests)

Der Rutschknoten SxSxS hielt im Pulsationstest im Durchschnitt beim Industriestandard 553 Zyklen (Mittelwert aus 24 Tests), bevor der Knoten aufging, beim mit dem Triblockcopolymer beschichteten Faden im Durchschnitt 1000 Zyklen (Mittelwert aus 18 Tests)

Der mit dem Triblockcopolymer beschichtete Faden ist beim SxSxS (Rutschknoten) sicherer als der Standardfaden.

**Beispiel 19**: Beschichtung von multifilem chirurgischem Nahtmaterial mit dem erfindungsgemäßen Polymer aus Beispiel 3 (6,0 % Lösung) unter Zugabe von 4 % Calciumstearat

60 g des Polymeren aus Beispiel 3 und 40 g Calciumstearat wurden in 900 g Xylol bei 140°C gelöst. Anschließend wurde die erkaltete Lösung mit einem starken Rührer zu einem dünnflüssigen Beschichtungsbad homogenisiert. Durch dieses Beschichtungsbad wurde bei einer Temperatur von 22°C ein Faden der Stärke USP 3/0 mit einer konstanten Geschwindigkeit von 20 m/min kurz durchgeführt und beschichtet. Nach Verlassen des Beschichtungsbades wurde der Faden bei ca. 160°C im Heizkanal getrocknet und aufgespult. Der Beschichtungsgehalt wurde mittels NMR zu 3,63 Gew.% bestimmt.

Der Rutschknoten SxSxS hielt im Streichtest im Durchschnitt beim Industriestandard 525 Zyklen (Mittelwert aus 12 Tests), bevor der Knoten aufging, beim mit dem Triblockcopolymer beschichteten Faden im Durchschnitt 1000 Zyklen (Mittelwert aus 6 Tests)

Der Rutschknoten SxSxS hielt im Pulsationstest im Durchschnitt beim Industriestandard 553 Zyklen (Mittelwert aus 24 Tests), bevor der Knoten aufging, beim mit dem Triblockcopolymer beschichteten Faden im Durchschnitt 858 Zyklen (Mittelwert aus 6 Tests)

Der mit dem Triblockcopolymer beschichtete Faden ist beim SxSxS (Rutschknoten) sicherer als der Standardfaden.

**Beispiel 20**: Beschichtung von multifilem chirurgischem Nahtmaterial mit dem erfindungsgemäßen Polymer aus Beispiel 9 (5,0 % Lösung) unter Zugabe von 2 % Calciumstearat

50 g des Polymeren aus Beispiel 9 und 20 g Calciumstearat wurden in 900 g Xylol bei 140°C gelöst. Anschließend wurde die erkaltete Lösung mit einem starken Rührer zu einem dünnflüssigen Beschichtungsbad homogenisiert. Durch dieses Beschichtungsbad wurde bei einer Temperatur von 22°C ein Faden der Stärke USP 3/0 mit einer konstanten Geschwindigkeit von 20 m/min kurz durchgeführt und beschichtet. Nach Verlassen des Beschichtungsbades wurde der Faden bei ca. 160°C im Heizkanal getrocknet und aufgespult. Der Beschichtungsgehalt wurde mittels NMR zu 3,18 Gew.% bestimmt.

Der Rutschknoten SxSxS hielt im Streichtest im Durchschnitt beim Industriestandard 525 Zyklen (Mittelwert aus 12 Tests), bevor der Knoten aufging, beim mit dem Triblockcopolymer beschichteten Faden im Durchschnitt 1000 Zyklen (Mittelwert aus 6 Tests)

Der Rutschknoten SxSxS hielt im Durchschnitt beim Industriestandard 553 Zyklen (Mittelwert aus 24 Tests), bevor der Knoten aufging, beim mit dem Triblockcopolymer beschichteten Faden im Durchschnitt 852 Zyklen (Mittelwert aus 6 Tests)

Der mit dem Triblockcopolymer beschichtete Faden ist beim SxSxS (Rutschknoten) sicherer als der Standardfaden.

**Patentansprüche**

1. Resorbierbares Blockcopolymer der Formel $X(BA)_n$ mit endständigen Hydroxylgruppen, wobei n 2,3 oder 4 ist, X ein Rest eines mehrwertigen Alkohols mit zwei bis vier Hydroxylgruppen ist, B ein Weichsegment aus Caprolacton, Trimethylericarbonat und gegebenenfalls Glykolid ist, wobei die Gesamtmenge an Trimethylencarbonat und Caprolacton im Weichsegment bei 80 bis 100 Gew.-% und die von Glykolid bei 0 bis 20 Gew.-% liegt, A ein Hartsegment aus Caprolacton ist und wobei das Gesamtgewichtsverhältnis von Caprolacton zu Trimethylencarbonat im Blockpolymer im Bereich von 65:35 bis 90:10 liegt, das Blockpolymer eine Schmelzenthalpie von mindestens 32 J/g, insbesondere im Bereich von 32 bis 58 J/g besitzt, wobei die Schmelzenthalpie bei einem Gesamtcaprolactonanteil von 65 Gew.-% eher im unteren Teil des Bereichs und bei einem Caprolactonanteil von 90 Gew.-% eher im oberen Teil des Bereichs liegt.

2. Blockpolymer nach Anspruch 1, **dadurch gekennzeichnet, dass** die Schmelzenthalpie bei einem Caprolactonanteil im Blockpolymer von 65 bis 80 Gew.-% bei 32 bis 45 J/g oder höher und bei einem Caprolactonanteil von 75 bis 90 Gew.-% bei 45 bis 58 J/g liegt.

3. Blockcopolymer nach Anspruch 1 oder 2, **dadurch gekennzeichnet, dass** der Anteil an Caprolacton-Caprolacton-Bindungen im Hartsegment bei mehr als 80 %, insbesondere mehr als 90 %, bevorzugt bei 92 bis 98 %, liegt, wobei der Anteil an Caprolacton-Caprolacton-Bindungen bei einem Caprolacton zu Trimethylencarbonat Verhältnis von 65:35 bis 75:25 eher bei 80 bis 92 % und bei einem Verhältnis von 75:25 bis 90:10 eher bei 92 bis 98 % liegt.

4. Blockcopolymer nach einem der vorhergehenden Ansprüche, **dadurch gekennzeichnet, dass** die Schmelzenthalpie bei Blockpolymeren mit 50 Gew.-% Hartsegmentanteil bei mindestens 32 J/g, bei Blockpolymeren mit Hartsegmentanteil von 60 Gew.-% bei mindestens 37 J/g, bei Hartsegmentanteilen von 70 Gew.-% bei mindestens 45 J/g und bei Hartsegmentanteilen von 70 bis 75 Gew.-% bei 45 bis 58 J/g, liegt.

5. Blockcopolymer nach einem der vorhergehenden Ansprüche, **dadurch gekennzeichnet, dass** ein Restmonomergehalt im Blockpolymer unter 2 Gew.-% liegt.

6. Blockcopolymer nach einem der vorhergehenden Ansprüche, **dadurch gekennzeichnet, dass** es einen Glasübergangspunkt besitzt, der maximal bei -45 °C, insbesondere zwischen -45 °C und -60 °C, liegt.

7. Blockcopolymer nach einem der vorhergehenden Ansprüche, **dadurch gekennzeichnet, dass** es einen Schmelzpeak (DSC) besitzt, der im Bereich von 38 bis 57 °C liegt.

8. Blockcopolymer nach einem der vorhergehenden Ansprüche, **dadurch gekennzeichnet, dass** der mehrwertige Alkohol ein Alkohol aus der Gruppe Ethylenglykol, Diethylenglykol, Hexandiol, Glycerin und Pentaerytrit ist.

9. Formkörper, umfassend ein Blockcopolymer nach einem der vorhergehenden Ansprüche.

10. Medizinisches Produkt, insbesondere Nahtmaterial, umfassend ein Blockcopolymer nach einem der Ansprüche 1 bis 8, vorzugsweise eine Beschichtung aus dem Blockcopolymer nach einem der Ansprüche 1 bis 8, insbesondere in Kombination mit Magnesium- und/oder Calciumsalz von Fettsäuren oder Fettsäureestern, insbesondere solchen der Stearinsäure, wobei das Verhältnis aus Polymer zu Fettsäuresalz zwischen 25 : 75 und 75 : 25 Gew.%, vorzugsweise zwischen 40 : 60 und 60 : 40 Gew.%, beträgt.

11. Verwendung des Blockcopolymers nach einem der Ansprüche 1 bis 8 als Trägermaterial für medizinische Wirkstoffe, insbesondere Drug-delivery-Material, und/oder als Trägermaterial für die gesteuerte Geweberegeneration.

12. Resorbierbares chirurgisches Nahtmaterial, im Wesentlichen bestehend aus dem Blockcopolymer nach einem der Ansprüchen 1 bis 8.

13. Nervenleitschienen, bei denen mindestens eine äußere Membran aus dem Blockcopolymer nach einem der Ansprüche 1 bis 8 gebildet ist.

14. Verfahren zur Herstellung des Blockcopolymers nach einem der Ansprüche 1 bis 8, bei dem in einer ersten Stufe Trimethylencarbonat, Caprolacton und gegebenenfalls Glykolid unter Verwendung eines zwei- bis vierwertigen Alkohols als Initiator zu einem vollständig amorphen hydroxy-terminierten Prepolymer, das das Weichsegment bildet, polymerisiert werden und danach in einer zweiten Stufe das Caprolacton unter Ausbildung des Hartsegments und Bildung des Blockcopolymers anpolymerisiert wird, **dadurch gekennzeichnet, dass** die Temperatur während der zweiten Stufe bei maximal 150 °C gehalten wird.

15. Verfahren nach Anspruch 14, **dadurch gekennzeichnet, dass** die Reaktion der ersten Stufe bei einer Temperatur von 180 °C bis 220 °C, insbesondere 200 °C bis 210 °C, durchgeführt wird.

16. Verfahren nach einem der Ansprüche 14 oder 15, **dadurch gekennzeichnet, dass** die Reaktion der zweiten Stufe bei einer Temperatur von 130 bis 150 °C, insbesondere 135 bis 150 °C, durchgeführt wird.

17. Verfahren nach einem der Ansprüche 14 bis 16, **dadurch gekennzeichnet, dass** die Reaktion der zweiten Stufe ohne vorherige Isolierung des Weichsegments nach Absenkung der Temperatur auf maximal 150 °C durchgeführt wird.

## Claims

1. Absorbable block copolymer of the formula $X(BA)_n$ with terminal hydroxyl groups, where n is 2, 3 or 4, X is a radical of a polyhydric alcohol having two to four hydroxyl groups, B is a soft segment formed from caprolactone, trimethylene carbonate and optionally glycolide, wherein the total amount of trimethylene carbonate and caprolactone in the soft segment is 80 to 100 wt% and that of glycolide is 0 to 20 wt%, A is a hard segment formed from caprolactone and wherein the total weight ratio of caprolactone to trimethylene carbonate in the block polymer is in the range from 65:35 to 90:10, the block polymer has a melt enthalpy of at least 32 J/g, especially in the range from 32 to 58 J/g, wherein the melt enthalpy is in the lower part of the range at a total caprolactone fraction of 65 wt% and in the upper part of the range at a caprolactone fraction of 90 wt%.

2. Block polymer according to Claim 1, **characterized in that** the melt enthalpy is 32 to 45 J/g or higher at a caprolactone fraction in the block polymer of 65 to 80 wt% and 45 to 58 J/g at a caprolactone fraction of 75 to 90 wt%.

3. Block copolymer according to Claim 1 or 2, **characterized in that** the proportion of caprolactone-caprolactone bonds in the hard segment is more than 80%, especially more than 90% and preferably in the range from 92 to 98%, wherein the proportion of caprolactone-caprolactone bonds is 80 to 92% at a caprolactone to trimethylene carbonate ratio of 65:35 to 75:25 and in the range from 92 to 98% at a ratio of 75:25 to 90:10.

4. Block copolymer according to any preceding claim, **characterized in that** the melt enthalpy is at least 32 J/g in the case of block polymers with 50 wt% hard segment fraction, at least 37 J/g in the case of block polymers with hard segment fraction of 60 wt%, at least 45 J/g in the case of hard segment fractions of 70 wt% and in the range from 45 to 58 J/g in the case of hard segment fractions of 70 to 75 wt%.

5. Block copolymer according to any preceding claim, **characterized in that** a residual monomer content in the block copolymer is below 2 wt%.

6. Block copolymer according to any preceding claim, **characterized in that** it has a glass transition point which is not more than -45°C and especially between -45°C and -60°C.

7. Block copolymer according to any preceding claim, **characterized in that** it has a melting peak (DSC) in the range from 38 to 57°C.

8. Block copolymer according to any preceding claim, **characterized in that** the polyhydric alcohol is an alcohol from the group ethylene glycol, diethylene glycol, hexanediol, glycerol and pentaerythritol.

9. Shaped article comprising a block copolymer according to any preceding claim.

10. Medical product, especially suture material, comprising a block copolymer according to any one of Claims 1 to 8, preferably a coating of the block copolymer according to any one of Claims 1 to 8, especially in combination with magnesium salt and/or calcium salt of fatty acids or fatty acid esters, especially those of stearic acid, wherein the ratio of polymer to fatty acid salt is between 25:75 and 75:25 wt% and preferably between 40:60 and 60:40 wt%.

11. Use of the block copolymer according to any one of Claims 1 to 8 as carrier material for active medical ingredients, especially drug delivery material, and/or as carrier material for controlled tissue regeneration.

12. Absorbable surgical suture material consisting essentially of the block copolymer according to any one of Claims 1 to 8.

13. Nerve guidance rails wherein at least one outer membrane is formed of the block copolymer according to any one of Claims 1 to 8.

14. Method for preparing the block copolymer according to any one of Claims 1 to 8 wherein, in a first step, trimethylene carbonate, caprolactone and optionally glycolide are polymerized by using a two-hydric to four-hydric alcohol as initiator to form a wholly amorphous hydroxyl-terminated prepolymer which forms the soft segment and then, in a second step, the caprolactone is polymerized thereonto to form the hard segment and the block copolymer, **characterized in that** the temperature during the second step is maintained at not more than 150°C.

15. Method according to Claim 14, **characterized in that** the reaction of the first step is carried out at a temperature of 180°C to 220°C and especially 200°C to 210°C.

16. Method according to either Claim 14 or 15, **characterized in that** the reaction of the second step is carried out at a temperature of 130 to 150°C and especially 135 to 150°C.

17. Method according to any one of Claims 14 to 16, **characterized in that** the reaction of the second step is carried out without isolating the soft segment beforehand, by lowering the temperature to not more than 150°C.

**Revendications**

1. Copolymère en bloc résorbable de formule $X(BA)_n$ à groupes hydroxy en bout de chaîne, n étant 2, 3 ou 4, X étant un reste d'un alcool polyhydrique comportant deux à quatre groupes hydroxy, B étant un segment souple à base de caprolactone, carbonate de triméthylène et éventuellement glycolide, la quantité totale de carbonate de triméthylène et caprolactone dans le segment souple étant de 80 à 100 % en poids et celle de glycolide étant de 0 à 20 % en poids, A étant un segment rigide à base de caprolactone et le rapport pondéral total de la caprolactone au carbonate de triméthylène dans le polymère séquencé se situant dans la plage allant de 65:35 à 90:10, le polymère en bloc présentant une enthalpie de fusion d'au moins 32 J/g, en particulier dans la plage de 32 à 58 J/g, l'enthalpie de fusion se situant, pour une proportion totale de caprolactone de 65 % en poids, plutôt dans la partie inférieure de la plage et, pour une proportion de caprolactone de 90 % en poids, plutôt dans la partie supérieure de la plage.

2. Copolymère en bloc selon la revendication 1, **caractérisé en ce que** l'enthalpie de fusion se situe, pour une proportion totale de caprolactone de 65 à 80 % en poids dans le polymère en bloc, à 32 à 45 J/g ou plus et, pour une proportion de caprolactone de 75 à 90 % en poids, à 45 à 58 J/g.

3. Copolymère en bloc selon la revendication 1 ou 2, **caractérisé en ce que** la proportion de liaisons caprolactone-caprolactone dans le segment rigide est de plus de 80 %, en particulier de plus de 90 %, de préférence de 92 à 98 %, la proportion de liaisons caprolactone-caprolactone se situant, pour un rapport de caprolactone à carbonate de triméthylène de 65:35 à 75:25, plutôt à 80 à 92 % et, pour un rapport de 75:25 à 90:10, plutôt à 92 à 98 %.

4. Copolymère en bloc selon l'une quelconque des revendications précédentes, **caractérisé en ce que** l'enthalpie de fusion se situe, pour des polymères en bloc ayant une proportion de segments rigides de 50 % en poids, à au moins 32 J/g, pour des polymères en bloc ayant une proportion de segments rigides de 60 % en poids, à au moins 37 J/g, pour des proportions de segments rigides de 70 % en poids, à au moins 45 J/g, et pour des proportions de segments rigides de 70 à 75 % en poids, à 45 à 58 J/g.

5. Copolymère en bloc selon l'une quelconque des revendications précédentes, **caractérisé en ce qu'**une teneur en monomères résiduels du polymère en bloc est inférieure à 2 % en poids.

6. Copolymère en bloc selon l'une quelconque des revendications précédentes, **caractérisé en ce qu'**il présente une température de transition vitreuse qui est au maximum de -45 °C, en particulier comprise entre -45 °C et -60 °C.

7. Copolymère en bloc selon l'une quelconque des revendications précédentes, **caractérisé en ce qu'**il présente un pic de fusion (DSC) qui se situe dans la plage de 38 à 57 °C.

8. Copolymère en bloc selon l'une quelconque des revendications précédentes, **caractérisé en ce que** l'alcool polyhydrique est un alcool choisi dans le groupe constitué par l'éthylèneglycol, le diéthylèneglycol, l'hexanediol, le glycérol et le pentaérythritol.

9. Corps moulé, comprenant un copolymère en bloc selon l'une quelconque des revendications précédentes.

10. Produit médical, en particulier matériel de suture, comprenant un copolymère en bloc selon l'une quelconque des revendications 1 à 8, de préférence un revêtement à base du copolymère en bloc selon l'une quelconque des revendications 1 à 8, en particulier en association avec un sel de magnésium et/ou de calcium d'acides gras ou d'esters d'acides gras, en particulier ceux de l'acide stéarique, le rapport du polymère au sel d'acide gras étant compris entre 25:75 et 75:25 % en poids, de préférence entre 40:60 et 60:40 % en poids.

11. Utilisation du copolymère en bloc selon l'une quelconque des revendications 1 à 8 en tant que matériau véhicule pour substances actives médicales, en particulier Drug-delivery-Material, et/ou en tant que matériau véhicule pour la régénération tissulaire programmée.

12. Matériel de suture chirurgical résorbable, consistant essentiellement en le copolymère en bloc selon l'une quelconque des revendications 1 à 8.

13. Guides de nerfs, dans lesquels au moins une membrane externe est constituée du copolymère en bloc selon l'une quelconque des revendications 1 à 8.

14. Procédé pour la préparation du copolymère en bloc selon l'une quelconque des revendications 1 à 8, dans lequel dans une première étape on polymérise du carbonate de triméthylène, de la caprolactone et éventuellement du glycolide en utilisant comme amorceur un alcool dihydrique à tétrahydrique, pour obtenir un prépolymère totalement amorphe à terminaison hydroxy, qui constitue le segment souple, et ensuite dans une deuxième étape on polymérise encore la caprolactone avec élaboration du segment rigide et formation du copolymère en bloc, **caractérisé en ce que** pendant la deuxième étape on maintient la température à au maximum 150 °C.

15. Procédé selon la revendication 14, **caractérisé en ce que** la réaction de la première étape est effectuée à une température de 180 °C à 220 °C, en particulier de 200 °C à 210 °C.

**16.** Procédé selon la revendication 14 ou 15, **caractérisé en ce que** la réaction de la deuxième étape est effectuée à une température de 130 à 150 °C, en particulier de 135 à 150 °C.

**17.** Procédé selon l'une quelconque des revendications 14 à 16, **caractérisé en ce qu'**on effectue la réaction de la deuxième étape sans isolement préliminaire du segment souple, après abaissement de la température jusqu'à 150 °C au maximum.

**Abb.1**: $^1$H-NMR-Spektrum eines Prepolymers (Weichsegment) der Zusammensetzung CL/T=30/70 Gewichtsprozent

**Abb.2**: ¹H-NMR-Spektrum eines ABA-Blockcopolymers der Zusammensetzung CL/T = 79/21 Gewichtsprozent mit einem Weichsegmentanteil von 30 Gewichtsprozent

**Abb.3**: Erzielbare Schmelzpunkte (Peakmaximum im DSC) und Schmelzenthalpien der erfindungsgemäßen Blockcopolymere (Weichsegmentzusammensetzung CL/T = 30/70; 100% Hartsegmentanteil = Polycaprolacton)

**Abb.4**: DSC-Plot (Ausschnitt) eines erfindungsgemäßen ABA-Blockcopolymers mit der Zusammensetzung CL/T = 79/21 Gesichtsprozent und einem Weichsegmentanteil von 30 Gewichtsprozent

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0835895 A
- US 6048947 A
- EP 0441322 A1
- US 475619 A
- EP 0626404 A2
- US 68811 A
- US 204721 A
- EP 1181941 A
- US 6706058 B
- US 4201216 A
- US 4705820 A
- US 5371176 A
- US 4857602 A
- US 20030147934 A1

**In der Beschreibung aufgeführte Nicht-Patentliteratur**

- **Dunnen.** *J. Biomater. Res.,* 1997, vol. 36 (3), 337-346
- **Pego.** *J. Mater. Sci. Mater. Med.,* 2003, vol. 14 (9), 767-773
- **Pego.** *J. Biomater. Sci. Polym. Ed.,* 2001, vol. 12, 35-53
- **Bei Lietz.** *Biotechnology and Bioengineering,* 2006, vol. 93 (1), 99-109